(19) Europäisches Patentamt
European Patent Office
Office européen des brevets

(11) EP 3 936 112 A1

(12) EUROPEAN PATENT APPLICATION

(43) Date of publication:
**12.01.2022 Bulletin 2022/02**

(21) Application number: **20305776.5**

(22) Date of filing: **07.07.2020**

(51) Int Cl.:
***A61K 9/00*** *(2006.01)* ***A61K 9/16*** *(2006.01)*
***A61K 9/19*** *(2006.01)* ***A61K 9/50*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Occlugel**
**78350 Jouy en Josas (FR)**

(72) Inventors:
- **BEILVERT, Anne**
**91300 MASSY (FR)**
- **CORUS, Emeline**
**91640 JANVRY (FR)**
- **BEDOUET, Laurent**
**72000 LE MANS (FR)**
- **MOINE, Laurence**
**92210 SAINT CLOUD (FR)**

(74) Representative: **Regimbeau**
**20, rue de Chazelles**
**75847 Paris Cedex 17 (FR)**

# (54) HYDROPHILIC DEGRADABLE MICROSPHERES FOR DELIVERING BUPRENORPHINE

(57) The invention relates to a composition comprising an effective amount of buprenorphine or a pharmaceutically acceptable salt thereof, at least one hydrophilic degradable microsphere comprising a crosslinked matrix, and a pharmaceutically acceptable carrier for administration by injection, the crosslinked matrix being based on at least a) between 10 % and 90 % of hydrophilic monomer of general formula (I); b) between 0.1 and 30% mol of a cyclic monomer having an exo-methylene group of formula (II); and c) between 5 % and 90 % of one degradable block copolymer cross-linker, wherein the degradable block copolymer crosslinker is linear or star-shaped and presents ($CH_2$=($CR_{11}$))-groups at all its extremities and wherein the degradable block copolymer crosslinker has a partition coefficient P of between 0.50 and 11.20. The invention also relates to such a composition for use for preventing and/or treating moderate to severe pain, post-surgical pain and/or opioid use disorders.



Printed by Jouve, 75001 PARIS (FR)

## Description

### FIELD OF THE INVENTION

The present invention relates to hydrophilic degradable microsphere for delivering buprenorphine or a pharmaceutically acceptable salt thereof. In particular, the present invention relates to composition comprising an effective amount of buprenorphine or a pharmaceutically acceptable salt thereof and hydrophilic degradable microspheres. The present invention also relates to said composition for use for preventing and/or treating moderate to severe pain, in particular post-surgical pain.

### TECHNICAL BACKGROUND

It is well-known in the art that pain, in particular acute pain, can be treated with opioids such as buprenorphine. Buprenorphine has a 20-40 times higher potency than morphine. The slow dissociation of buprenorphine from the receptor results in a long duration effect. Buprenorphine has been used for years as an analgesic for treatment of moderate to severe pain, in particular in post-surgical patients. Buprenorphine is thus well-known for promoting a strong analgesic action. Buprenorphine has also been used for years for treating opiate addiction.

Oral or sublingual buprenorphine formulations are well-known by the one skilled in the art. These formulations are approved for once or twice administration per day, and may optionally be approved for an administration three times per day. However, the lack of compliance and the possible misuse of these formulations lead to the need of new methods of administering buprenorphine.

In addition, oral formulation of buprenorphine has some disadvantages such as poor gastrointestinal absorption and low systemic availability leading to ineffective pain relief. After oral uptake, buprenorphine undergoes an extensive first-pass through the intestinal wall and hepatic metabolism, resulting in low and variable systemic exposures. Sublingual and transdermal methods of drug administration avoid the first-pass metabolism as they bypass the gastrointestinal tract, thereby improving bioavailability.

The sublingual formulations can improve bioavailability over oral administration through a rapid absorption resulting in high systemic exposure and rapidly attained plasma levels. However, inadvertent swallowing of a part of the sublingual dose can induce a pre-systemic first-pass elimination inducing variation of plasma concentration and large inter-subject variability in drug absorption (Kapil et al; 2013. J Pain Symptom Manage. 46:65-75).

Buprenorphine transdermal patch has been approved for the sustained administration of buprenorphine for the treatment of moderate-to-severe cancers and non-cancer related pain, including post-operative, and musculoskeletal pain such osteoarthritis. Transdermal patches of buprenorphine provide more stable plasma concentrations, controlled and sustained delivery. The seven-day transdermal patches are capable of delivering a constant treatment rate for one week. The delivery rates of transdermal patches are 5 - 10 or 20 μg/h. These devices yield buprenorphine plasma concentration in a therapeutic threshold with a high level of patient satisfaction. For example, the Butrans® patch at 20 μg/h allows a rapid plasma concentration of 200 pg/mL after four days before a slow decrease up to day 7 until this replacement by another patch (Kapil et al; 2013. J Pain Symptom Manage. 46:65-75). With patches, a stable buprenorphine level can be achieved with the first application and is maintained with repeated weekly applications. However, the use of transdermal patches induces secondary effects, the most common are nausea, constipation, fatigue, dizziness and headache even if the plasmatic concentrations are low (50-70 pg/mL) (Al-Tawil et al; 2013. Eur J Clin Pharmacol. 69:143-149).

Parenteral formulations are also well-known by the one skilled in the art but are poorly tolerated or even prohibited in patients with specific indications and may lead to central side effects. To avoid the secondary effects of systemic applied opioids, their local injection is proposed in order to obtain a peripheral analgesic effect. For example, buprenorphine was injected directly in the operative wounds after surgery in the management of post-surgical pain (Metha et al, 2011, J Anaesthesiol Clin Pharmacol. 27:211-4). Buprenorphine (2 μg/kg) was injected in addition to bupivacaine in the wounds after nephrectomy in 20 patients. The use of rescue analgesics during the first day after surgery was significantly reduced compared to patients treated locally only with bupiviacaine. Direct injection of opioids in the joint cavity after arthroscopy alleviates the post-operative pain (Kalso et al; 2002; Pain. 98; 269-75). For instance, the intra-articular injection of buprenorphine (100 μg) after knee arthroscopy reduced the post-operative pain up to 6 h after surgery. The pain score was lower compared to the control group at 6 h treated with intra-muscular injection of buprenorphine (Varrassi et al; 1999. Acta Anaesthesiol Scand. 43: 51-5).

The rationale behind the local injections of opioids (morphine, buprenorphine) in painful tissues is based on the recent discovery of opioid receptors on the peripheral nerve endings of afferent neurons, the nociceptors. Opioid receptors were first observed on nerve fibres in knee joint of cat (Russell 1987, Neurosci Lett. 23:107-12.) and observations made by stein et al (1991; N Engl J Med. 325:1123-6) indicate that the opioid receptors in the human knee joint are functional. Intra-articular injection of morphine (1 mg) alleviates pain in patients after arthroscopy surgery more efficiently than

intravenous injection of morphine. As a result, peripheral opioid receptors located in the skin, joints and viscera can be targeted for effective analgesia without the central effects seen with systemic opioid treatment. Local delivery of opioids in wounded tissues is a solution to inhibit the pain at its source (Machelska & Celik. 2018. Frontiers in Pharmacology. Vol 9).

The peripheral opioid system allows the development of novel strategies for pain treatment, by divorcing analgesic action from unwanted central side effects. Different solutions for peripheral analgesia are investigated such the development of new opioid drugs with a low diffusion in brain or drug delivery systems (DDS) for local delivery of opioids in peripheral tissues (Machelska & Celik. 2018. Frontiers in Pharmacology. Vol 9). However, a meta-analysis (26 clinical studies, 1531 patients, 13 different surgical interventions) reveals that peripherally applied opioids for acute postoperative pain are ineffective (Nielsen et al; 2015. Acta Anaesthesiol Scand. 59:830-45). The analgesic effect of peripherally applied opioids seems to depend on the presence of preoperative inflammation. The analgesic effect of peripheral opioids has been reported to increase linearly between 6 and 24 h of inflammation in animal studies (Zhou et al; 1998. J Pharmacol Exp Ther. 286:1000-6), indicating that up-regulation of peripheral opioid receptors may be delayed from the induction of inflammation caused by surgery. Significant antalgic effects of locally applied opioids are reported when a pre-existing inflammation was documented before the surgery, for example with patients who underwent hemorrhoidectomy (Tegon et al; 2009, Tech. Coloproctol, 13: 219-24). It is supposed that locally applied opioids are probably removed from the tissue by drainage before the expression of their receptors had occurred. A solution would be a local post-operative injection of injectable DDS that would gradually elute opioids during expression of opioids receptors on sensory neurons in the hours following the surgery.

In the art, several injectable drug delivery systems (DDS) for buprenorphine are reported. Several DDS were designed for the opioid addiction management in patients (review in Rosenthal and Goradia; 2017. Drug Des Devel Ther. 11: 2493-2505). Simon et al; 2006 (Addiction. 101:420-32) developed long-acting biodegradable microspheres composed of poly(L-lactide-co-glycolide) microspheres thanks to sustained release of buprenorphine. After subcutaneous implantation the buprenorphine peaks in plasma after 2-3 days and reach undetectable level after 6 weeks. A rod-shaped implant (26 mm × 2.5 mm) buprenorphine (80 mg or 90 mg) (Probuphine®) was made in polymeric matrix composed of ethylene vinyl acetate. After sub-dermal implantation, buprenorphine was delivered at a steady rate for 6 months (White et al; 2009. Drug Alcohol Depend. 2009. 103:37-43). More recently, a novel long-term buprenorphine delivery system to control opioid dependence, maned RBP-6000, was synthesized. Buprenorphine (300 mg) is mixed in PLGA dissolved in N-methyl-pyrrolidone (NMP). After subcutaneous injection, the NMP diffuses out of the polymer and the polymer precipitates, trapping the buprenorphine inside and forming a solid deposit *in situ.* The deposit releases buprenorphine for one month by diffusion as the polymer degrades (Nasser et al; 2014. Clin Pharmacokinet. 53:813-24). In WO 2017/147285, buprenorphine is encapsulated in poly(D,L-lactide-co-glycolide) microspheres (20-40 μm) for a plasmatic sustained release ranging between weeks and months according to the molecular weight of the degradable poly(D,L-lactide-co-glycolide). A different delivery strategy to deliver buprenorphine is based on the CAM2038 technology, where lipids in contact with aqueous media self-assemble into reversed-phase "water-in-oil" non-lamellar liquid crystal nanoparticle gels (FluidCrystal®; Camurus AB, Lund, Sweden). After injection, through a small 23-gauge needle, the depot in contact with interstitial aqueous fluid transforms into a viscous liquid-crystal gel phase that elutes buprenorphine as the depot degrades. The release is sustained for 1 week up to 1 month (Frost et al; 2019. Addiction. 114: 1416-26). In addition, one composition (Buprenorphine SR™) is described for pain management of small laboratory animals after subcutaneous injection. The delivery system is a biodegradable copolymer (50:50 molar ratio) of DL-lactide and ε-caprolactone dissolved in a biocompatible organic solvent for a release of buprenorphine for 3 days. Buprenorphine is entrapped after injection upon polymer precipitation, the drug release occurred by diffusion (Foley et al; 2011. J Am Assoc Lab Anim Sci. 50:198-204).

The inventors have noticed the interest represented by buprenorphine delivery systems for the sustained release of buprenorphine. According to the art, it appears a need for novel delivery systems of buprenorphine easily injectable, which degrade quickly and without organic solvent in their composition.

## SUMMARY OF THE INVENTION

The inventors have discovered hydrophilic degradable microspheres that may be used as biocompatible drug carrier for local delivery and that present affinity with the active ingredient buprenorphine.

The inventors surprisingly found that degradable hydrophilic microspheres composed of a crosslinked hydrogel displays strong affinity for buprenorphine allowing an efficient loading in few minutes and the subsequent sustained release. The present invention offers the possibility after mini-invasively injection, a local sustained release of low dose of buprenorphine after surgical intervention in the aim to get a pain relief for several days. This approach would allow when the patient returns home to reduce the use of various analgesics, including opiates avoiding the diversion of prescription.

The inventors have thus discovered a buprenorphine delivery system that is free of organic solvent, that presents a tuneable degradation time from day to months, that is easy to load (in water, at room temperature), that allows a

sustained and complete drug release and that avoids immediate burst and intense inflammatory reaction.

The buprenorphine delivery system of the invention allows an extended and local release of buprenorphine. For example, the composition of the invention allows to extend post-surgical pain management period by local delivery of low amounts of buprenorphine. Such a delivery avoids central side effects while allowing an extended duration of peripheral analgesia without the need of several administrations of opioids. For example, the composition of the invention may be useful for pain relief for a few days with a single injection.

In a first aspect, the invention relates to a composition comprising an effective amount of buprenorphine or a pharmaceutically acceptable salt thereof, at least one hydrophilic degradable microsphere comprising a crosslinked matrix, and a pharmaceutically acceptable carrier for administration by injection, the crosslinked matrix being based on at least:

a) between 10 % and 90 % of hydrophilic monomer of general formula (I):

$$(CH_2{=}CR_1)\text{-}CO\text{-}D \qquad (I)$$

wherein:

- D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)m$ or $-(CH_2)_m\text{-}NR_5R_6$ with m being an integer from 1 to 30;
- $R_1$, $R_2$, $R_3$, $R_4$, $R_5$ and $R_6$ are, independently of one another, hydrogen atom or a $(C_1\text{-}C_6)$alkyl group;

b) between 0.1 and 30% mol of a cyclic monomer having an exo-methylene group of formula (II):

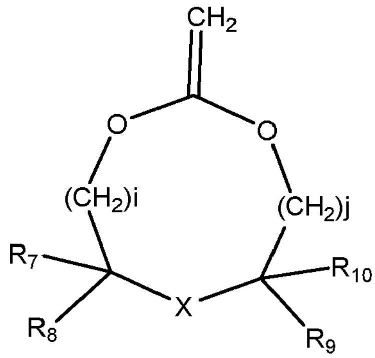

(II)

wherein:

- $R_7$, $R_8$, $R_9$ and $R_{10}$ are, independently of one another, hydrogen atom, a $(C_1\text{-}C_6)$alkyl group or an aryl group;
- i and j are independently of one another an integer chosen between 0 and 2; and
- X is a single bond or an oxygen atom;

and

c) between 5 % and 90 % of one degradable block copolymer cross-linker, wherein the degradable block copolymer crosslinker is linear or star-shaped and presents $(CH_2{=}(CR_{11}))$-groups at all its extremities, each $R_{11}$ being independently of one another hydrogen atom or a $(C_1\text{-}C_6)$alkyl group, and wherein the degradable block copolymer crosslinker has a partition coefficient P of between 0.50 and 11.20, or a hydrophobic/hydrophilic balance R between 1 and 20;

the percentages of the monomers a) to c) being expressed in moles relative to the total number of moles of the monomers.

In a second aspect, the invention relates to the composition of the invention for use for preventing and/or treating moderate to severe pain; post-surgical pain, advantageously for locally treating post-surgical pain; or opioid use disorders.

In a third aspect, the invention relates to the hydrophilic degradable microsphere of the invention for use for delivering an effective amount of buprenorphine or a pharmaceutically acceptable salt thereof to a subject in need thereof.

In a fourth aspect, the invention relates to a pharmaceutical kit comprising:

i) at least one hydrophilic degradable microsphere of the invention in association with a pharmaceutically acceptable carrier for administration by injection;

ii) an effective amount of buprenorphine or a pharmaceutically acceptable salt thereof; and
iii) optionally an injection device,

the hydrophilic degradable microsphere and the buprenorphine or a pharmaceutically acceptable salt thereof being packed separately.

## DETAILED DESCRIPTION OF THE INVENTION

*Definition*

In the context of the invention, by the expression "matrix based on" is meant a matrix comprising the mixture and/or the product of the reaction between the starting components used for the heterogeneous medium polymerisation of this matrix, preferably only the product of the reaction between the different starting components used for this matrix, some of which may be intended to react or may react with each other or with their close chemical environment, at least in part, during the different phases of the process of manufacture of the matrix, in particular during a polymerisation stage.
Thus, the starting components are the reagents intended to react together during the polymerization of the matrix. The starting components are therefore introduced into a reaction mixture optionally additionally comprising a solvent or a mixture of solvents and/or other additives such as at least one salt and/or at least one polymerization initiator and/or at least one stabilizer such as PVA.
In the context of the present invention, the reaction mixture comprises at least the monomers a), b), c) as defined in the claims and in this description, optionally a polymerization initiator such as, for example, t-butyl peroxide, benzoyl peroxide, azobiscyanovaleric acid (also called 4,4'-Azobis(4-cyanopentanoic acid)), AIBN (azobisisobutyronitrile), or 1,1'- Azobis(cyclohexane carbonitrile) or one or more photo-initiators such as 2-Hydroxy-4'-(2-hydroxyethoxy)-2-methylpropiophenone (106797-53-9); 2-Hydroxy-2-methylpropiophenone (Darocur® 1173, 7473-98-5); 2,2-Dimethoxy-2-phenylacetophenone (24650-42-8); 2,2-Dimethoxy-2-phenyl acetophenone (Irgacure®, 24650-42-8) or 2-Methyl-4'-(methylthio)-2-morpholinopropiophenone (Irgacure®, 71868-10-5), and at least one solvent, preferably a solvent mixture comprising an aqueous solvent and an organic solvent such as an apolar aprotic solvent, for example a water/toluene mixture.
Thus, according to the present invention, the matrix is at least based on the monomers a), b) and c) as defined in the claims and in this description, these compounds thus being starting components.
Thus, in the present description, expressions such as "the [starting component X] is added to the reaction mixture in an amount of between YY% and YYYY%" and "the cross-linked matrix is based on [starting component X] in an amount of between YY% and YYYY%" are interpreted in a similar manner. Similarly, expressions such as "the reaction mixture comprises at least [starting component X]" and "the cross-linked matrix is based on at least [starting component X]" are interpreted in a similar manner.
In the context of the invention, "organic phase" of the reaction mixture means the phase comprising the organic solvent and the compounds soluble in said organic solvent, in particular the monomers, and the polymerization initiator.
In the context of the invention, the terms "($C_X$-$C_Y$)alkyl group" mean a saturated monovalent hydrocarbon chain, linear or branched, containing X to Y carbon atoms, X and Y being integers between 1 and 36, preferably between 1 and 18, in particular between 1 and 6. Examples are methyl, ethyl, propyl, isopropyl, butyl, isobutyl, sec-butyl, tert-butyl, pentyl or hexyl groups.
In the context of the invention, the terms "aryl group" and "($C_X$-$C_Y$)aryl" mean an aromatic hydrocarbon group, preferably having X to Y carbon atoms, and comprising one or more adjacent rings, X and Y being integers between 5 and 36, preferably between 5 and 18, in particular between 5 and 10. Examples are phenyl or naphthyl groups.
In the context of the invention, the terms "partition coefficient P" mean the ratio of concentrations of a compound in a mixture of two immiscible solvents at equilibrium: water and 1-octanol. This ratio is therefore a comparison of the solubilities of the solute in these two liquids. Hence the octanol/water partition coefficient measures how hydrophilic (octanol/water ratio < 1) or hydrophobic (octanol/water > 1) a compound is. Partition coefficient P may be determined by measuring the solubilities of the compound in water and in 1-octanol and by calculating the ratio solubility in octanol / solubility in water. Partition P may also be determined *in silico.*
In the context of the invention, the hydrophobic/hydrophilic balance R of the degradable crosslinkers is quantified by the ratio of the number of hydrophobic units to the number of hydrophilic units according the following equation:

$$R = \frac{N_{hydrophobic\ units}}{N_{hydrophilic\ units}}$$

with N being an integer and representing the number of unit(s).

For example, for the crosslinkers that can be used in the present invention, R is:

$$R = \frac{N_{CHlactide} + N_{CH3lactide} + N_{CH2glycolide} + 5 \times N_{CH2caprolactone}}{N_{EO\ unit}}$$

with N being an integer and representing the number of unit(s).

In the context of the invention, the terms "degradable microsphere" mean that the microsphere is degraded or cleaved by hydrolysis in a mixture of degradation products composed of low-molecular-weight compounds and water-soluble polymer chains having molecular weights below the threshold for renal filtration of 50 kg $mol^{-1}$. Compared to non-degradable microspheres, no ester hydrolysable bond is present with the crosslinker.

In the context of the invention, the expression "between X and Y" (wherein X and Y are numerical value) means a range of numerical values in which the limits X and Y are inclusive.

In the context of the invention, the expression "immediate release (IR)" of an active ingredient means the rapid release of the active ingredient from the formulation to the location of delivery as soon as the formulation is administered.

In the context of the invention, the expression "extended-release" of an active ingredient means either the "sustained-release (SR)" or the "controlled-release (CR)" of active ingredients from the formulation to the location of delivery at a predetermined rate for an extended period of time and maintaining a constant active ingredient level for this period of time with minimum side effects. The controlled-release (CR) differs from the sustained-release (SR) in that CR maintains drug release over a sustained period at a constant rate whereas SR maintains drug release over a sustained period but not at a constant rate.

In the context of the invention, the expression "sustained-release" of an active ingredients means an extended-release (as defined above) of an active ingredient from the formulation to the location of delivery in order to maintain for a certain predetermined time the drug in tissue of interest at therapeutic concentrations by means of an initial dose portion

In the context of the invention, the expression "controlled-release (CR)" of an active ingredient means an extended-release (as defined above) of an active ingredient from the formulation to the location of delivery that provides some control of temporal or spatial nature, or both.

For the purpose of the invention, the term "pharmaceutically acceptable" is intended to mean what is useful to the preparation of a pharmaceutical composition, and what is generally safe and non toxic, for a pharmaceutical use.

The term « pharmaceutically acceptable salt » is intended to mean, in the framework of the present invention, a salt of a compound which is pharmaceutically acceptable, as defined above, and which possesses the pharmacological activity of the corresponding compound. Such salts comprise:

(1) hydrates and solvates,
(2) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(3) salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

*Microsphere*

According to the present invention, the hydrophilic degradable microsphere comprises a crosslinked matrix that is based on at least:

a) between 10 and 90% of hydrophilic monomer of general formula (I):

$(CH_2{=}CR_1)\text{-}CO\text{-}D$ (I)

wherein:

- D is O-Z or NH-Z, Z being $(C_1\text{-}C_6)$alkyl group, $-(CR_2R_3)_m-CH_3$, $-(CH_2-CH_2-O)_m-H$, $(CH_2-CH_2-O)_m-CH_3$, $-C(R_4OH)m$ or $-(CH_2)_m-NR_5R_6$ with m being an integer from 1 to 30;

- $R_1$, $R_2$, $R_3$, $R_4$, $R_5$ and $R_6$ are, independently of one another, hydrogen atom or a $(C_1-C_6)$alkyl group;

b) between 0.1 and 30% mol of a cyclic monomer having an exo-methylene group of formula (II):

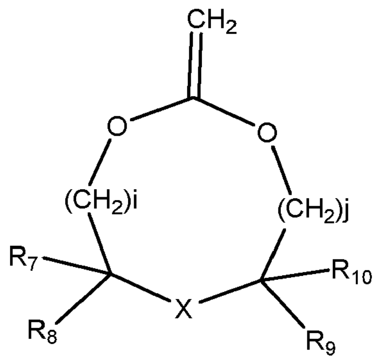

(II)

wherein:

- $R_7$, $R_8$, $R_9$ and $R_{10}$ are, independently of one another, hydrogen atom, a $(C_1-C_6)$alkyl group or an aryl group;
- i and j are independently of one another an integer chosen between 0 and 2; and
- X is a single bond or an oxygen atom;

and

c) between 5 and 90% of one degradable block copolymer cross-linker, wherein the degradable block copolymer crosslinker is linear or star-shaped and presents $(CH_2=(CR_{11}))$-groups at all its extremities, each $R_{11}$ being independently of one another hydrogen atom or a $(C_1-C_6)$alkyl group, and wherein the degradable block copolymer crosslinker has a partition coefficient P between 0.5 and 11.2 or a hydrophobic/hydrophilic balance R between 1 and 20;

the percentages of the monomers a) to c) being expressed in moles relative to the total number of moles of the monomers.

The components a) the hydrophilic monomer of general formula (I), b) the cyclic monomer having an exo-methylene group of formula (II), and c) the degradable block copolymer cross-linker, are thus starting components of the crosslinked matrix.

In the context of the invention, the hydrophilic degradable microsphere is a swellable degradable (i.e. hydrolyzable) cross-linked polymer in the form of spherical particle having a diameter after swelling ranging between 20 μm and 1200 μm. In particular the polymer of the invention is constituted of at least one chain of polymerized monomers a), b) and c) as defined above.

In the context of the invention, a polymer is swellable if it has the capacity to absorb liquids, in particular water. The expression "size after swelling" means thus that the size of the microspheres is considered after the polymerization and sterilization steps that take place during their preparation.

Advantageously, the microsphere of the invention has a diameter after swelling of between 20 μm and 100 μm, 40 μm and 150 μm, 100 μm and 300 μm, 300 μm and 500 μm, 500 μm and 700 μm, 700 μm and 900 μm or 900 μm and 1200 μm, as determined by optical microscopy. Microspheres are advantageously small enough in diameter to be injected through needles, catheters or microcatheters with internal diameters ranging from a few hundred micrometres to more than one millimetres.

In the context of the invention, the terms "hydrophilic monomer" mean a monomer having a high affinity for water, i.e. tending to dissolve in water, to mix with water, to be wetted by water, or capable of swelling in water after polymerization.

The hydrophilic monomer a) is of general formula (I):

$$(CH_2=CR_1)\text{-CO-D} \qquad \text{(I)}$$

wherein:

- D is O-Z or NH-Z, Z being $(C_1-C_6)$alkyl group, $-(CR_2R_3)_m-CH_3$, $-(CH_2-CH_2-O)_m-H$, $(CH_2-CH_2-O)_m-CH_3$, $-C(R_4OH)m$ or $-(CH_2)_m-NR_5R_6$ with m being an integer from 1 to 30;

- $R_1$, $R_2$, $R_3$, $R_4$, $R_5$ and $R_6$ are, independently of one another, hydrogen atom or a ($C_1$-$C_6$)alkyl group.

Advantageously, the hydrophilic monomer a) is selected from the group consisting of sec-butyl acrylate, n-butyl acrylate, t-butyl acrylate, t-butyl methacrylate, methylmethacrylate, N-dimethylaminoethyl(methyl)acrylate, N,N-dimethylaminopropyl-(meth)acrylate, t-butylaminoethyl (methyl)acrylate, N,N-diethylaminoacrylate, acrylate terminated poly(ethylene oxide), methacrylate terminated poly(ethylene oxide), methoxy poly(ethylene oxide) methacrylate, butoxy poly(ethylene oxide) methacrylate, acrylate terminated poly(ethylene glycol), methacrylate terminated poly(ethylene glycol), methoxy poly(ethylene glycol) methacrylate, butoxy poly(ethylene glycol) methacrylate; advantageously acrylate terminated poly(ethylene glycol), methacrylate terminated poly(ethylene glycol), methoxy poly(ethylene glycol) methacrylate, butoxy poly(ethylene glycol) methacrylate.
More advantageously, the hydrophilic monomer a) is poly(ethylene glycol) methyl ether methacrylate.
In the context of the invention, the hydrophilic monomer a) is advantageously present in the reaction mixture in an amount of between 10% and 90%, preferably from between 30% and 80 %, by mole, relative to the total number of moles of the monomers.
In the context of the invention, component b) is a cyclic monomer having an exo-methylene group of formula (II) as defined above, wherein:

- $R_7$, $R_8$, $R_9$ and $R_{10}$ are, independently of one another, hydrogen atom, a ($C_1$-$C_6$)alkyl group or an aryl group;
- i and j are independently of one another an integer chosen between 0 and 2;
- X is a single bond or an oxygen atom.

Advantageously, component b) is a cyclic monomer having an exo-methylene group of formula (II) as defined above, wherein:

- $R_7$, $R_8$, $R_9$ and $R_{10}$ are, independently of one another, hydrogen atom or a ($C_5$-$C_7$)aryl group;
- i and j are independently of one another an integer chosen between 0 and 2;
- X is a single bond or an oxygen atom.

Advantageously, component b) is a cyclic monomer having an exo-methylene group of formula (II) as defined above, wherein:

- $R_7$, $R_8$, $R_9$ and $R_{10}$ are, independently of one another, hydrogen atom or a ($C_5$-$C_7$)aryl group;
- i and j are independently of one another an integer chosen between 0 and 1;
- X is a single bond or an oxygen atom.

Advantageously, the component b) is selected from the group consisting of 2-methylene-1,3-dioxolane, 2-methylene-1,3-dioxane, 2-methylene-1,3-dioxepane, 2-Methylene-1,3,6-Trioxocane and derivatives thereof, in particular benzo derivatives and phenyl substituted derivatives, advantageously from the group consisting of 2-methylene-1,3-dioxolane, 2-methylene-1,3-dioxane, 2-methylene-1,3-dioxepane, 2-methylene-4-phenyl-1,3-dioxolane, 2-Methylene-1,3,6-Trioxocane and 5,6-benzo-2-methylene-1,3dioxepane, more advantageously from the group consisting of 2-methylene-1,3-dioxepane, 5,6-benzo-2-methylene-1,3dioxepane and 2-Methylene-1,3,6-Trioxocane. More advantageously, the component b) is 2-methylene-1,3-dioxepane or 2-Methylene-1,3,6-Trioxocane.
In the context of the invention, the cyclic monomer b) having an exo-methylene group of general formula (II) is advantageously present in the reaction mixture in an amount of between 0.1 % and 30 %mol, preferably from between 1% and 20 %, and in particular between 1% and 10% by mole, relative to the total number of moles of the monomers.
In the context of the invention, component c) is a degradable block copolymer crosslinker, wherein the degradable block copolymer crosslinker is linear or star-shaped and presents ($CH_2$=($CR_{11}$))-groups at all its extremities, each $R_{11}$ being independently of one another hydrogen atom or a ($C_1$-$C_6$)alkyl group.
In the context of the invention, the degradable block copolymer crosslinker has a partition coefficient P of between 0.50 and 11.20, advantageously between 3.00 and 9.00. Or the degradable block copolymer crosslinker has a hydrophobic/hydrophilic balance R between 1 and 20, advantageously between 3 and 15.
The hydrophobicity of the crosslinker, and thus the partition coefficient P or the hydrophobic/hydrophilic balance R of the crosslinker, influences the loading of the microsphere in buprenorphine and then the release of the buprenorphine. A crosslinker having a partition coefficient P of more than 1.90 or a hydrophobic/hydrophilic balance R more than 2 leads to an optimal release of buprenorphine, in particular because it prevents the immediate release of a large part of the buprenorphine.
As intended therein, the expression "copolymer cross-linker" is intended to mean that the copolymer contains a functional group containing a double bond at least two of its extremities in order to link together several polymer chains.

The cross-linker c) as defined above is linear or star-shaped (advantageously from 3 to 8 arms) and it presents ($CH_2$=($CR_{11}$))-groups at all its extremities, each $R_{11}$ being independently of one another hydrogen atom or a ($C_1$-$C_6$)alkyl group. Advantageously, the crosslinker c) presents ($CH_2$=($CR_{11}$))-CO- or ($CH_2$=($CR_{11}$))-CO-O groups at all its extremities, each $R_{11}$ being independently of one another hydrogen atom or a ($C_1$-$C_6$)alkyl group. Advantageously, the $R_{11}$ are identical and are H or a ($C_1$-$C_6$)alkyl group.

The cross-linker c) as defined above is linear and it presents ($CH_2$=($CR_{11}$))-groups at both its extremities, each $R_{11}$ being independently of one another hydrogen atom or a ($C_1$-$C_6$)alkyl group. Advantageously, the crosslinker c) presents ($CH_2$=($CR_{11}$))-CO- or ($CH_2$=($CR_{11}$))-CO-O groups at both its extremities, each $R_{11}$ being independently of one another hydrogen atom or a ($C_1$-$C_6$)alkyl group. Advantageously, the $R_{11}$ are identical and are H or a ($C_1$-$C_6$)alkyl group.

Advantageously, the crosslinker c) is of general formula (IIIa), (IIIb) or (IIIc) as follows:

$(CH_2=CR_{11})\text{-}CO\text{-}X_n\text{-}PEG_p\text{-}X_k\text{-}CO\text{-}(CR_{11}=CH_2)$ (IIIa);

$(CH_2=CR_{11})\text{-}CO\text{-}O\text{-}X_n\text{-}PEG_p\text{-}X_k\text{-}O\text{-}CO\text{-}(CR_{11}=CH_2)$ (IIIb)

$PEG_p\text{-}(X_n\text{-}O\text{-}CO\text{-}(CR_{11}=CH_2))_z$ (IIIc);

wherein:

- each $R_{11}$ is independently of one another hydrogen atom or a ($C_1$-$C_6$)alkyl group;
- X independently represents PLA, PGA, PLGA, PCL or PLAPCL;
- n, k and p respectively represent the degree of polymerization of X, and PEG, n and k independently being integers from 1 to 150, and p being an integer from 1 to 100;
- z represent the number of arms of the PEG molecule being integers from 3 to 8.

Advantageously, when the crosslinker c) is of general formula (IIIc), n may be identical or different in each arm of the PEG.

In the context of the invention:

- PEG means polyethylene glycol of formula (IVa):

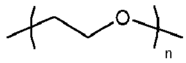
(IVa);

- PLA means poly-lactic acid (also named poly-lactide) of formula (IVb):

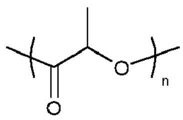
(IVb);

- PGA means poly-glycolic acid (also named poly-glycolide) of formula (IVc):

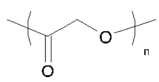
(IVc);

- PLGA means poly-lactic-glycolic acid and comprises both lactide and glycolide units, the degree of polymerization is the sum of the number of lactide and glycolide units;
- PCL means poly(caprolactone) of formula (IVd):

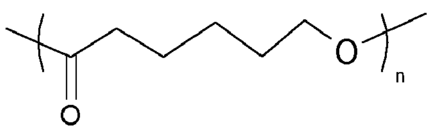
(IVd);

and

- PLAPCL means poly-lactic acid poly-caprolactone and comprises both lactide and caprolactone units, the degree of polymerization is the sum of the number of lactide and caprolactone units.

Advantageously, the crosslinker c) is of general formula (IIIa), (IIIb) or (IIIc), in particular (IIIa) or (IIIb), as defined above, wherein X represents PLAPCL or PCL. More advantageously, the crosslinker c) is of general formula (IIIa), (IIIb) or (IIIc), in particular (IIIa) or (IIIb) wherein X represents PCL.

Advantageously, the crosslinker c) is of general formula (IIIa), (IIIb) or (IIIc), in particular (IIIa) or (IIIb), as defined above, wherein n and k independently being integers from 1 to 150, and p being an integer from 1 to 100.

Advantageously, the crosslinker c) is of general formula (IIIa), (IIIb) or (IIIc), in particular (IIIa) or (IIIb), as defined above, wherein the $R_{11}$ are identical and are H or a $(C_1-C_6)$alkyl group.

Advantageously, the crosslinker c) is selected from the group consisting of compounds of general formula (IIIa), (IIIb) or (IIIc), in particular (IIIa) or (IIIb), as defined above, wherein:

- X= PLA, n + k =12 and p=13 (such as PEG13-PLA12);
- X= PLAPCL, n + k =10 and p=13 (such as PEG13-LA8-Cl2 or PEG13-LA7-Cl3);
- X = PLAPCL, n + k = 9 and p=13 (such as PEG13-LA4-Cl5);
- X = PLAPCL, n + k = 8 and p=13 (such as PEG13-LA2-Cl6);
- X = PCL; n + k = 8 and p=13 (such as PEG13-PCl8);
- X =PCL, n + k = 10 and p=4 (such as PEG4-PCL10); or
- X =PCL, n + k = 12 and p=2 (such as PEG2-PCL12).

Within the definitions of the crosslinker c) above, the polyethylene glycol (PEG) has a length of 100 to 10 000 g/mol, preferably 100 to 2 000 g/mol, more preferably 100 to 1 000 g/mol.

In the context of the invention, the crosslinker c) is advantageously present in the reaction mixture in an amount of between 5 % and 90 %mol, preferably from between 5% and 60 %, relative to the total number of moles of the monomers.

According to the invention, the crosslinked matrix of the hydrophilic degradable microsphere is advantageously further based on a chain transfer agent d).

For the purposes of this invention, "transfer agent" means a chemical compound having at least one weak chemical bond. This agent reacts with the radical site of a growing polymer chain and interrupts the growth of the chain. In the chain transfer process, the radical is temporarily transferred to the transfer agent which restarts growth by transferring the radical to another polymer or monomer.

Advantageously, the chain transfer agent d) is selected from the group consisting of monofunctional or polyfunctional thiols, alkyl halides, transition metal salts or complexes and other compounds known to be active in free radical chain transfer processes such as 2,4-diphenyl-4-methyl-1-pentene. More advantageously, the chain transfer agent is a cycloaliphatic or aliphatic, thiol preferably having from 2 to 24 carbon atoms, more preferably between 2 and 12 carbon atoms, and having or not a further functional group selected from the groups amino, hydroxy and carboxy. Advantageously, the chain transfer agent d) is selected from the group consisting of thioglycolic acid, 2-mercaptoethanol, dodecane thiol and hexane thiol.

According to the invention, the chain transfer agent is advantageously present in the reaction mixture in an amount of, for example, from 0.1 to 10%, preferably from 2 to 5 % by mole, relative to the number of moles of hydrophilic monomer a).

In a particular aspect of the invention, the crosslinked matrix is only based on starting components a), b), c) and optionally d), as defined above and in the contents abovementioned, no other starting component are thus added to the reaction medium. It is thus clear that the sum of the above-mentioned contents of monomers (a), (b) and (c) must be equal to 100 %.

According to another aspect of the invention, the crosslinked matrix is advantageously further based on at least one ionised or ionisable monomer e) of general formula (V):

$$(CH_2{=}CR_{12})\text{-M-E} \qquad (V),$$

wherein:

- $R_{12}$ is hydrogen atom or a $(C_1-C_6)$alkyl group;
- M is a single bond or a divalent radical having 1 to 20 carbon atoms, advantageously a single bond;
- E is a ionised or ionisable group being advantageously selected from the group consisting of-COOH, -COO-, $-SO_3H$, $-SO_3^-$, $-PO_4H_2$, $-PO_4H^-$, $-PO_4^{2-}$, $-NR_{13}R_{14}$, and $-NR_{15}R_{16}R_{17}^+$; $R_{13}$, $R_{14}$, $R_{15}$, $R_{16}$ et $R_{17}$ being independently of one another hydrogen atom or a $(C_1-C_6)$alkyl group.

In the context of the invention, an ionised or ionisable group is understood to be a group which is charged or which may be in charged form (in the form of an ion), i.e. which carries at least one positive or negative charge, depending on the pH of the medium. For example, the COOH group may be ionised in the $COO^-$ form, and the $NH_2$ group may be ionised in the form of $NH_3^+$.

The introduction of an ionised or ionisable monomer into the reaction media makes it possible to increase the hydrophilicity of the resulting microspheres, thereby increasing the swelling rate of said microspheres, further facilitating their injection via catheters and microcatheters. In addition, the presence of an ionised or ionisable monomer improves the loading of active substances into the microsphere.

In an advantageous embodiment, the ionised or ionisable monomer e) is a cationic monomer, advantageously selected from the group consisting of -(methacryloyloxy)ethyl phosphorylcholine, 2-(dimethylamino)ethyl (meth)acrylate, 2-(diethylamino)ethyl (meth)acrylate and 2-((meth)acryloyloxy)ethyl] trimethylammonium chloride, more advantageously the cationic monomer is diethylamino)ethyl (meth)acrylate. Advantageously, the ionised or ionisable e) is present in the reaction mixture in an amount of between 0 % and 30 % by mole, advantageously between 1 % and 30 % by mole, preferably from between 10% and 15 % by mole, relative to the total number of moles of the monomers.

In another advantageous embodiment, the ionised or ionisable monomer e) is an anionic monomer advantageously selected from the group consisting of acrylic acid, methacrylic acid, 2-carboxyethyl acrylate, 2-carboxyethyl acrylate oligomers, 3-sulfopropyl (meth)acrylate potassium salt and 2-(methacryloyloxy)ethyl]dimethyl-(3-sulfopropyl)ammonium hydroxide, more advantageously, the anionic monomer is acrylic acid. Advantageously, the ionised or ionisable monomer e) is present in the reaction mixture in an amount of between 0 % and 30 % by mole, advantageously between 1 % and 30 % by mole, preferably from between 10% and 15 % by mole, relative to the total number of moles of the monomers.

Advantageously, the ionised or ionisable monomer e) is acrylic acid and is advantageously present in the reaction mixture in an amount of between 0 % and 30 % by mole, advantageously between 1 % and 30 % by mole, preferably from between 10% and 15 % by mole, relative to the total number of moles of the monomers.

The microsphere of the invention can be readily synthesized by numerous methods well-known to the one skilled in the art. By way of example, the microsphere of the invention can be obtained by direct or inverse suspension polymerization as described below and, in the Examples, or by microfluidic.

A direct suspension may proceed as follows:

(1) stirring or agitating a mixture comprising

(i) at least the starting components a), b) and c) as defined above;
(ii) a polymerization initiator present in amounts ranging from 0.1 to approximately 2 parts per weight per 100 parts by weight of the monomers;
(iii) a surfactant in an amount no greater than about 5 parts by weight per 100 parts by weight of the monomers, preferably no greater than about 3 parts by weight and most preferably in the range of 0.5 to 1.5 parts by weight; and
(iv) water to form an oil in water suspension;

and

(2) polymerizing the starting components.

In such a direct suspension polymerization, the surfactant may be selected from the group consisting of hydroxyethylcellulose, polyvinyl alcohol (PVA), polyvinylpyrrolidone, polyethylene oxide, polyethylene glycol and Polysorbate 20 (Tween® 20).

An inverse suspension may proceed as follows:

(1) stirring or agitating a mixture comprising:

(i) at least the starting components a), b) and c) as defined above;
(ii) a polymerization initiator present in amounts ranging from 0.1 to approximately 2 parts per weight per 100 parts by weight of the monomers;
(iii) a surfactant in an amount no greater than about 5 parts by weight per 100 parts by weight of the monomers, preferably no greater than about 3 parts by weight and most preferably in the range of 0.5 to 1.5 parts by weight; and
(iv) oil to form a water in oil suspension;

and

(2) polymerizing the starting components.

In such a reverse suspension process, the surfactant may be selected from the group consisting of sorbitan esters such as sorbitan monolaurate (Span® 20), sorbitan monopalmitate (Span® 40), sorbitan monooleate (Span® 80), and sorbitan trioleate (Span® 85), hydroxyethyl cellulose, mixture of glyceryl stearate and PEG stearate (Arlacel®) and cellulose acetate.

In the above processes, the polymerization initiator may include t-butyl peroxide, benzoyl peroxide, azobiscyanovaleric acid (also known as 4,4'-Azobis(4-cyanopentanoic acid)), AIBN (azobisisobutyronitrile), or 1,1' Azobis (cyclohexane carbonitrile) or one or more photo-initiators such as 2-Hydroxy-4'-(2-hydroxyethoxy)-2-methylpropiophenone (106797-53-9); 2-Hydroxy-2-methylpropiophenone (Darocur® 1173, 7473-98-5); 2,2-Dimethoxy-2-phenylacetophenone (24650-42-8); 2,2-dimethoxy-2-phenyl acetophenone (Irgacure®, 24650-42-8) or 2-Methyl-4'-(methylthio)-2-morpholinopropiophenone (Irgacure®, 71868-10-5).

Further, the oil may be selected from paraffin oil, silicone oil and organic solvents such as hexane, cyclohexane, ethyl acetate or butyl acetate.

Loading may proceed by numerous methods well-known to one of skill in the art such as passive adsorption (swelling of the polymer into a drug solution) or by ionic interaction.

In order to increase the drug loading and control the rate of drug release, a concept consists to introduce certain chemical moieties into the polymer backbone that are capable of interacting with the drug via non covalent interactions. Examples of such interactions include electrostatic interactions (described above), hydrophobic interactions, $\pi$-$\pi$ stacking, and hydrogen bonding, among others.

*Drug*

In the context of the invention, the composition comprises an effective amount of buprenorphine or a pharmaceutically acceptable salt thereof.

Advantageously, the buprenorphine is in the form of a free base or a pharmaceutically acceptable salt thereof such as buprenorphine hydrochloride. Advantageously, the buprenorphine is in the form of hydrochloride salt.

Advantageously, in the composition of the invention, the buprenorphine or the pharmaceutically acceptable salt thereof is loaded/absorbed onto the microsphere as defined above by non-covalent interactions. This particular way of entrapping drugs or prodrugs is called physical entrapment.

Loading of buprenorphine or the pharmaceutically acceptable salt thereof onto the microsphere of the invention may be proceeded by numerous methods well-known to the one skilled in the art such as preloading buprenorphine after the microsphere synthesis. Buprenorphine or the pharmaceutically acceptable salt thereof in solution is mixed with the degradable microspheres over a predetermined time and then the mixture is freeze-dried.

Advantageously, the composition of the invention comprises between 0.1 and 50 mg/ml of buprenorphine or a pharmaceutically acceptable salt thereof, more advantageously between 0.5 and 10 mg/ml.

Advantageously, the composition of the invention releases the buprenorphine or a pharmaceutically acceptable salt thereof without a burst, less 20 % during the first day, followed by a constant delivery rate between 5 % and 15 % of initial loading every day for 1 to 30 days.

Advantageously, after implantation in living organisms, the composition of the invention releases the buprenorphine or a pharmaceutically acceptable salt thereof in the plasma without a burst during the first hour following implantation (skin, joint, viscera, muscle). Concentration of buprenorphine could remain in the therapeutic range for 1 to 7 days, advantageously for 1 to 15 days, preferably for 1 to 30 days, the therapeutic range being between 1 ng/ml to 8 ng/ml, or preferably without plasma detection when the loading value of buprenorphine is low (< 1mg/mL) for local delivery in order to obtain a peripheral analgesia without plasmatic exposition.

*Composition*

In the context of the invention, the composition comprises an effective amount of buprenorphine or a pharmaceutically acceptable salt thereof, at least one hydrophilic degradable microsphere as defined above, and a pharmaceutically acceptable carrier for administration by injection.

The buprenorphine or a pharmaceutically acceptable salt thereof and the hydrophilic degradable microsphere are as defined above.

According to the invention, the pharmaceutically acceptable carrier is intended for administration of the buprenorphine or a pharmaceutically acceptable salt thereof by injection and is advantageously selected in the group consisting in water for injection, starch, hydrogel, polyvinylpyrrolidone, polysaccharide, hyaluronic acid ester, contrast

agent, and plasma.

The formulations may be administered by intramuscular, subcutaneous or intra-articular injection. The formulations of degradable microspheres are syrangable, the microsphere size and distribution are shown in Example 7 for example. This enables the administration in a needle that is from between 21 and 34 gauge.

The composition of the invention can also contain a buffering agent, a preservative, a gelling agent, a surfactant, or mixtures thereof. Advantageously, the pharmaceutically acceptable carrier is saline or water for injection.

The composition of the invention allows the extended-release, in particular the controlled-release, of buprenorphine or a pharmaceutically acceptable salt thereof over a period ranging from a few hours to a few months. Advantageously, the composition of the invention allows the controlled-release of buprenorphine or a pharmaceutically acceptable salt thereof for 1 day to 7 days, advantageously for 1 to 15 days, more advantageously for 1 to 30 days.

The composition of the invention allows the temporal control and the sustained-release as defined above, for example by modulating the nature and the contents of monomers a) and/or c).

The invention also relates to the composition as defined above, for use for preventing and/or treating moderate to severe pain.

The invention also relates to the composition as defined above, for use for preventing and/or treating post-surgical pain, in particular for locally treating post-surgical pain.

The invention also relates to the composition as defined above, for use for preventing and/or treating opioid use disorders such as opioid dependence, opioid tolerance and opioid withdrawal symptoms.

The invention also relates to a method for preventing and/or treating moderate to severe pain, comprising administering to a subject in need thereof an effective amount of the composition as defined above.

The invention also relates to a method for preventing and/or treating post-surgical pain, in particular for locally treating post-surgical pain, comprising administering, advantageously locally administering, to a subject in need thereof an effective amount of the composition as defined above.

The invention also relates to a method for preventing and/or treating opioid use disorders such as opioid dependence, opioid tolerance and opioid withdrawal symptoms, comprising administering to a subject in need thereof an effective amount of the composition as defined above.

The invention also relates to the use of the composition as defined above for the manufacturing of a drug for preventing and/or treating moderate to severe pain.

The invention also relates to the use of the composition as defined above for the manufacturing of a drug for preventing and/or treating post-surgical pain, in particular for locally treating post-surgical pain.

The invention also relates to the use of the composition as defined above for the manufacturing of a drug for preventing and/or treating opioid use disorders such as opioid dependence, opioid tolerance and opioid withdrawal symptoms.

*Extemporaneous loading*

In a particular embodiment of the invention, the buprenorphine or the pharmaceutically acceptable salt thereof may be loaded extemporaneously on dry and sterile microsphere.

The invention thus also relates to a pharmaceutical kit comprising:

i) at least one hydrophilic degradable microsphere as defined above in association with a pharmaceutically acceptable carrier for administration by injection;
ii) an effective amount of buprenorphine or a pharmaceutically acceptable salt thereof; and
iii) optionally an injection device,

the hydrophilic degradable microsphere and the buprenorphine being packed separately.

In such an embodiment, the buprenorphine or the pharmaceutically acceptable salt thereof is advantageously intended to be loaded on the hydrophilic degradable microsphere just before the injection.

Advantageously, the buprenorphine or the pharmaceutically acceptable salt thereof is as defined above.

According to the present invention, "injection device" means any device for parenteral administration. Advantageously, the injection device is one or more syringes, which may be prefilled, and/or one or more catheters or microcatheters.

*Use of the microsphere*

The invention also relates to the hydrophilic degradable microsphere as defined above for use for the delivery, advantageously the controlled-delivery, of an effective amount of buprenorphine or a pharmaceutically acceptable salt thereof to a subject in need thereof.

The buprenorphine or a pharmaceutically acceptable salt thereof and the hydrophilic degradable microsphere are as defined above.

Advantageously, the sustained delivery of buprenorphine is over a period ranging from a few hours to a few months without burst, advantageously from 1 day to 7 days, advantageously for 1 to 15 days, more advantageously for 1 to 30 days.

The invention also relates to a method for delivering an effective amount of buprenorphine or a pharmaceutically acceptable salt thereof to a subject in need thereof, advantageously over a period ranging from a few hours to a few months without burst, advantageously from 1 day to 7 days, advantageously for 1 to 15 days, more advantageously for 1 to 30 days, comprising the administration of the hydrophilic degradable microsphere as defined above in association with a pharmaceutically acceptable carrier for administration by injection.

The examples which follow illustrate the invention without limiting its scope in any way.

DESCRIPTION OF FIGURES

Figure 1: In vitro release in PBS of buprenorphine after the extemporaneous loading on dry MS (50-100 μm). After the fast loading of buprenorphine on dry and sterile microspheres, the drug release follows the degradation of microspheres. A: release from MS3 (loading value = 0.89 mg/mL) which degraded in 9 days of incubation in PBS. B: release during 1 month from MS11 (loading value = 2.56 mg/mL) which degraded slowly in PBS (> 6 months).

Figure 2: Effect of crosslinker at 5 % on in vitro buprenorphine release in PBS. The buprenorphine loading is 0.84 ± 0.03 mg/mL (target 1 mg/mL). The duration of buprenorphine release in PBS increase with the MS degradation time, i.e according to the log P values of the degradable crosslinker.

Figure 3: Effect of crosslinker (at 5 %) on the initial burst release of buprenorphine during the first day at 37°C in PBS

Figure 4: Plasmatic concentrations of buprenorphine (BPN) after single subcutaneous injection of degradable microsphere loaded with BPN.

Figure 5: Tissular concentrations of buprenorphine (BPN) one month after single subcutaneous injection of degradable microsphere loaded with buprenorphine

Figure 6: Size distribution of sterile MS3 and MS4 after swelling in saline

Figure 7: Histology of MS3 after implantation in rabbit in several tissues (Fig. 7A: Subconjonctival implantation; Fig. 7B: Intra-articular (joint knee) injection; Fig. 7C: Intra-muscular injection ; Fig. 7D: Injection in gum). HES histology of tissues was done after 2-4 and 7 days of implantation.

Figure 8: Measure of in vivo degradation of MS3 in different tissues after implantation in rabbit. Percentage of MS degradation was determined after 2-4 and 7 days after implantation (100 μL of MS sediment in saline (0.9 % NaCl).

Figure 9: Measure of local inflammatory reaction during degradation of MS3 in different tissues after implantation in rabbit. Each slide was observed to identify where the inflammation was the most important in terms of thickness around the microspheres. This thickness was measured (μm) at the periphery of the implant and not within the implant at 3 different places. Percentage of different inflammatory cell types (macrophages (MP), giant cells (GC), polynuclears (PN), lymphocytes (LP), eosinophiles (EO)) present in the thickness was measured.

## EXAMPLES

### Example 1: Preparation of unloaded microsphere according to the invention

The starting components and their contents are summarized in Table 1a and in Table 1b. The tables 1a and 1b also summarize the main parameters.

| | | | MS1 | MS2 | MS3 | MS4 | MS5 | MS6 |
|---|---|---|---|---|---|---|---|---|
| | | Microsphere of 50-100 µm diameters | | | | | | |
| | | **MS number** | **MS1** | **MS2** | **MS3** | **MS4** | **MS5** | **MS6** |
| Process parameters | | Oil/Water ratio (V/V) | 1/11 | 1/11 | 1/11 | 1/11 | 1/11 | 1/11 |
| | | Stirring speed | 240 RPM | 240 RPM | 240 RPM | 240 RPM | 240 RPM | 240 RPM |
| | | PVA | 1 % | 1 % | 1 % | 1 % | 1 % | 1 % |
| | | NaCl | 3 % | 3 % | 3 % | 3 % | 3 % | 3 % |
| Organic phase | | Monomers mass / organic phase mass (%) | 56 % | 56 % | 56 % | 56 % | 56 % | 56 % |
| | | Toluene | 44% | 44% | 44% | 44% | 44% | 44% |
| | | Hexanethiol (% mole / PEGma mole) | 3 % | 3 % | 3 % | 3 % | 3 % | 3 % |
| | | AIBN (% weight/ organic phase weight) | 0.28% | 0.28% | 0.28% | 0.28% | 0.28% | 0.28% |
| | Phase monomer | m-PEGMA (% mole / total mole monomer) | 85 % | 85 % | 85 % | 85 % | 85% | 85 % |
| | | Crosslinker (% mole / total mole monomer) | 5 % of $PEG_{13}$-PLA | 5 % of $PEG_{13}$-$LA_8$-$Cl_2$ | 5 % of $PEG_{13}$-$LA_7$-$Cl_3$ | 5 % of $PEG_{13}$-$LA_4$-$Cl_5$ | 5 % of $PEG_{13}$-$PCl_8$ | 5 % of $PEG_2$-$PCL_{12}$ |
| | | 2-methylene-1,3-dioxepane (MDO) (% mole / total mole monomer) | 10 % | 10 % | 10 % | 10 % | 10 % | 10 % |
| | | Methacrylic acid (% mole / total mole monomer) | 0 % | 0 % | 0 % | 0 % | 0 % | 0 % |

**Table 1a. Formulations of microspheres according to the invention for buprenorphine loading**

| | | | Microsphere of 50-100 µm diameters | | | | | | |
|---|---|---|---|---|---|---|---|---|---|
| | | MS number | MS7 | MS8 | MS9 | MS10 | MS11 | MS12 | MS13 |
| Process parameters | | Oil/Water ratio (V/V) | 1/11 | 1/11 | 1/11 | 1/11 | 1/11 | 1/11 | 1/11 |
| | | Stirring speed | 240 RPM | 240 RPM | 240 RPM | 240 RPM | 240 RPM | 240 RPM | 240 RPM |
| | | PVA | 1 % | 1 % | 1 % | 1 % | 1 % | 1 % | 1 % |
| | | NaCl | 3 % | 3 % | 3 % | 3 % | 3 % | 3 % | 3 % |
| Organic phase | | Monomers mass / organic phase mass (%) | 56 % | 56 % | 56 % | 56 % | 56 % | 56 % | 56 % |
| | | Toluene | 44% | 44% | 44% | 44% | 44% | 44% | 44 % |
| | | Hexanethiol (% mole / PEGma mole) | 3 % | 3 % | 3 % | 3 % | 3 % | 3 % | 3 % |
| | | AIBN (% weight/ organic phase weight) | 0.28% | 0.28% | 0.28% | 0.28% | 0.28% | 0.28% | 0.28% |
| | Phase monomer | m-PEGMA (% mole / total mole monomer) | 75 % | 75 % | 75 % | 60 % | 60 % | 40 % | 40 % |
| | | Crosslinker (% mole / total mole monomer) | 15 % of $PEG_{13}$-$LA_7$-$Cl_3$ | 15 % of $PEG_{13}$-$PCl_8$ | 15 % of $PEG_2$-$PCL_{12}$ | 30 % of $PEG_{13}$-$LA_7$-$Cl_3$ | 30 % of $PEG_{13}$-$PCl_8$ | 50 % of $PEG_{13}$-$PCL_8$ | 50 % of $PEG_2$-$PCL_{12}$ |
| | | 2-methylene-1,3-dioxepane (MDO) (% mole / total mole monomer) | 10 % | 10 % | 10 % | 10 % | 10 % | 10 % | 10 % |
| | | Methacrylic acid (% mole / total mole monomer) | 0 % | 0 % | 0 % | 0 % | 0 % | 0 % | 0 % |

**Table 1b. Formulations of microspheres according to the invention for buprenorphine loading**

The aqueous phase solution (917 mL) containing 1 % polyvinyl alcohol (Mw= 13000-23000 g/mol), 3 % NaCl in deionized water is placed in a 1 $dm^3$ reactor and heated up to 50°C.

The organic phase is prepared in an Erlenmeyer. Briefly, toluene (36.9 g) and 2,2'-azobis(2-methylpropionitrile) (AIBN) (0.28% weight/ organic phase weight) are weighted. AIBN is introduced in another vial and solubilized in a volume fraction (≈ 30%) of the weighted toluene.

Then, degradable crosslinker is weighted in an Erlenmeyer. Polyethylene glycol methyl ether methacrylate (Mn= 300 g/mol) and 2-Methylene-1,3-dioxepane (MDO) are weighted and introduced into the erlenmeyer. Then the remaining volume of toluene is added to solubilize the monomers. Hexanethiol (3 % mol /mol of PEGma) is added to the Erlenmeyer. The AIBN solution in toluene is added to the erlenmeyer containing monomers. Finally, the organic phase has to be clear (monomer and initiator should be totally solubilized) without any aggregates before introduction into the aqueous phase.

The organic phase is poured into the aqueous phase at 50°C. Thereupon, stirring (240 rpm) is applied by using an impeller. After 4 minutes, the temperature is raised up to 80°C. After 8 hours, the stirring is stopped and microspheres were collected by filtration on a 40 μm sieve and washed extensively with acetone and water. Microspheres were then sieved with decreasing sizes of sieves (125, 100, 50 μm). MS in the size range 50-100 μm were collected for drug loading trials.

**Example 2: Loading of microspheres according to example 1 with buprenorphine hydrochloride (preloading after MS synthesis)**

After the sieving step, 1 mL of microspheres obtained in example 1 (size range 50-100 μm) was placed in 15 mL polypropylene vials and 3 mL of sodium bicarbonate (4mM) in water were added. The different MS compositions are given in table 2. After 5 min of mixing with microspheres at room temperature, buprenorphine hydrochoride (Sigma, PHR1729-50 MG) in water at 10 mg/mL was added (1 mg to 50 mg). Table 2 summarizes MS formulations used to investigate buprenorphine loading and sustained delivery.

Table 2: Crosslinker composition of degradable microspheres used for buprenorphine loading

| Crosslinker % | $PEG_{13}$-PLA | $PEG_{13}$-$LA_8$-$Cl_2$ | $PEG_{13}$-$LA_7$-$Cl_3$ | $PEG_{13}$-$LA_4$-$Cl_5$ | $PEG_{13}$-$PCl_8$ | $PEG_2$-$PCL_{12}$ |
|---|---|---|---|---|---|---|
| **5** | MS1 | MS2 | MS3 | MS4 | MS5 | MS6 |
| **15** | - | - | MS7 | - | MS8 | MS9 |
| **30** | - | - | MS10 | - | MS11 | - |
| **50** | - | - | - | - | MS12 | MS13 |

The loading step was done at room temperature for 2 h under stirring on a tube rotator (≈ 30 rpm). Then, the supernatants were removed for the measurement of unbound buprenorphine hydrochloride by fluorimetry ($\lambda_{ex}$ 280 nm, $\lambda_{Em}$ 354 nm). The amount of buprenorphine in supernatant was obtained by extrapolation from a standard curve (0.6 to 25 μg/mL), and the loaded dose was calculated by subtraction. The pellets were washed with 10 mL of water, then, the microsphere pellets were frozen-dried before e-beam sterilization (15 -25 kilograys). Table 3 summarizes buprenorphine loading for each MS formulation tested containing 5 % of crosslinker.

Table 3. Buprenorphine loading in the microspheres according to example 1. The loading of increasing amounts of buprenorphine is achievable with good yields.

| Test number | MS used | Buprenorphine loading (mg/ml) | Buprenorphine loading target (mg/mL) | Efficacy of buprenorphine loading (%) | Log P value for degradable crosslinkers |
|---|---|---|---|---|---|
| T1 | MS1 | 0.85 ± 0.031 | 1 | 86 | 0.5 |
| T2 | MS2 | 0.88 ± 0.021 | 1 | 88 | 1.8 |
| T3A | MS3 | 0.86 ± 0.05 | 1 | 86 | 3.2 |
| T3B | MS3 | 1.59 ± 0.004 | 2 | 79 | 3.2 |
| T3C | MS3 | 3.39 ± 0.28 | 4 | 84 | 3.2 |
| T4A | MS4 | 0.79 ± 0.019 | 1 | 79 | 4.1 |
| T4B | MS4 | 1.63 ± 0.004 | 2 | 81 | 4.1 |
| T4C | MS4 | 3.11 ± 0.16 | 4 | 78 | 4.1 |
| T5A | MS5 | 0.94 ± 0.003 | 1 | 94 | 6.5 |
| T6 | MS6 | 0.97 ± 0.003 | 1 | 97 | 11.2 |

Table 4 summarizes buprenorphine loading for MS formulation containing 5-15-30 or 50 % of degradable crosslinker.

Table 4. Buprenorphine loading in the microspheres according to example 1. Effect of the crosslinker composition and crosslinker concentration on buprenorphine loading.

| Test number | MS used | Buprenorphine loading (mg/ml) | Cross linker | % of degradable crosslinker | Buprenorphine loading target (mg/mL) | Efficacy of buprenorphine loading (%) | Log P of degradable crosslinker |
|---|---|---|---|---|---|---|---|
| T3A | MS3 | 0.86 ± 0.05 | $PEG_{13}$-$LA_7$-$Cl_3$ | 5 | 1 | 86 | 3.2 |
| T3D | MS7 | 0.95 ± 0.02 | | 15 | 1 | 95 | |
| T3E | MS10 | 0.99 ± 0.0007 | | 30 | 1 | 99 | |
| T5A | MS5 | 0.94 ± 0.003 | $PEG_{13}$-$PCl_8$ | 5 | 1 | 95 | 6.5 |
| T5B | MS5 | 37.8 ± 0.2 | | 5 | 50 | 75 | |
| T5C | MS8 | 0.98 ± 0.004 | | 15 | 1 | 98 | |
| T5D | MS8 | 3.87 ± 0.029 | | 15 | 4 | 97 | |
| T5E | MS8 | 38.2 ± 0.72 | | 15 | 50 | 76 | |
| T5F | MS11 | 0.98 ± 0.001 | | 30 | 1 | 98 | |
| T5G | MS12 | 3.90 ± 0.001 | | 50 | 4 | 96 | |
| T6A | MS6 | 0.97 ± 0.003 | $PEG_2$-$PCL_{12}$ | 5 | 1 | 97 | 11.2 |
| T6B | MS9 | 3.91 ± 0.013 | | 15 | 4 | 97 | |
| T6C | MS13 | 3.92 ± 0.025 | | 50 | 4 | 98 | |

**Example 3. Extemporaneous loading of buprenorphine hydrochloride on sterile microspheres according to example 1**

After the sieving step, a suspension of 1 mL of microspheres obtained in example 1 (size range 50-100 μm) in 15 mL of a solution containing 2.5 % (w/v) of mannitol and 4 mM of sodium bicarbonate was prepared. After homogenization, the pellet of microspheres was recovered, frozen-dried and sterilized by e-beam radiation (15-25 kilograys).
Then, buprenorphine hydrochloride (1 mg up to 4 mg) in solution in 3 mL of water were added to the sterile and dry microspheres.
The loading step was done at room temperature for 5 minutes under stirring on a tube rotator (≈ 30 rpm). Then, the supernatants were removed for the measurement of unbound buprenorphine by fluorimetry ($\lambda_{ex}$ 280 nm, $\lambda_{Em}$ 354 nm). The amount of buprenorphine was obtained by extrapolation from a standard curve (0.6 to 20 μg/mL). Then, PBS (25 mL) was added to the microspheres loaded with buprenorphine for the *in vitro* drug release experiment (37°C, 150 rpm). The release of buprenorphine was analysed by fluorimetry ($\lambda_{ex}$ 280 nm, $\lambda_{Em}$ 354 nm) (see Figure 1). Table 5 summarizes extemporaneous buprenorphine loading on dry and sterile MS

Table 5. Extemporaneous buprenorphine loading on dry and sterile microspheres

| Test number | MS used | Buprenorphine loading target (mg/mL) | Buprenorphine loading (mg/ml) | Efficacy of buprenorphine loading (%) |
|---|---|---|---|---|
| T3F | MS3 | 1 | 0.89 ± 0.031 | 89 |
| T3G | MS3 | 2 | 1.18 ± 0.66 | 59 |
| T3H | MS3 | 4 | 2.16 ± 1.14 | 54 |
| T5E | MS11 | 4 | 2.56 ± 0.15 | 64 |

**Example 4. Study of the *in vitro* release of buprenorphine from loaded microspheres according to example 2**

For sterile and dry microspheres loaded with buprenorphine hydrochloride, the swelling step of microspheres was performed for 10 min in 10 mL of water (Sigma W-3500). After the removal of water, 25 mL of PBS (Sigma P-5368; 10 mM phosphate buffered saline; NaCl 0,136 M; KCl 0,0027 M; pH 7.4 at 25 °C) were added.
Drug elution occurred at 37°C under shaking (150 rpm), the tubes were placed horizontally in the oven. Samples (2 mL) were withdrawn after 5h and every day during the first week and every 2-3 days for MS with slow degradation rate. At each sampling time, the medium was completely renewed with fresh PBS. The amounts of free buprenorphine in water and in PBS supernatants were determined by fluorometry (($\lambda_{ex}$ 280 nm, $\lambda_{Em}$ 354 nm).
Results are given in Table 6 and Figures 2 and 3.

Table 6: In vitro analysis of buprenorphine burst release during swelling of dry microspheres and subsequent incubation in PBS for 5 h (37°C). The burst release is low except for MS having a "PLA" crosslinker. The caprolactone moieties of the degradable crosslinkers are important to reduce the initial burst release of buprenorphine.

| Test number | | | % of buprenorphine release | | MS *in vitro* degradation time (days) |
|---|---|---|---|---|---|
| | MS used | Buprenorphine loading target (mg/mL) | During MS swelling | After 5h in PBS | |
| T1 | MS1 | 1 | X | 36 | 2 |
| T2 | MS2 | 1 | X | 14 | 6 |
| T3A | MS3 | 1 | 3.9 | 12.7 | 12 |
| T3B | MS3 | 2 | 5.7 | 17 | 12 |
| T3C | MS3 | 4 | 8 | 21 | 12 |
| T4A | MS4 | 1 | 3.5 | 12 | 50 |
| T4B | MS4 | 2 | 4.6 | 11 | 50 |
| T4C | MS4 | 4 | 7.5 | 15 | 50 |

(continued)

| Test number | MS used | Buprenorphine loading target (mg/mL) | % of buprenorphine release | | MS *in vitro* degradation time (days) |
|---|---|---|---|---|---|
| | | | During MS swelling | After 5h in PBS | |
| T5A | MS5 | 1 | X | X | 180 |
| T5B | MS5 | 50 | X | X | 180 |
| T6A | MS6 | 1 | X | X | > 6 months |
| T3D | MS7 | 1 | X | X | < 6 months |
| T5C | MS8 | 1 | X | X | > 6 months |
| T5D | MS8 | 4 | 4.7 | X | > 6 months |
| T5E | MS8 | 50 | X | X | > 6 months |
| T6B | MS9 | 4 | 3.4 | X | > 6 months |
| T3E | MS10 | 1 | X | X | > 6 months |
| T5C | MS11 | 1 | X | X | > 6 months |
| T5D | MS12 | 4 | 3.4 | X | > 6 months |
| T6C | MS13 | 4 | X | X | > 6 months |
| *X = not measured* | | | | | |

**Example 5. Study of the plasma buprenorphine concentration during the time following single subcutaneous injection in rabbit of degradable microspheres loaded with buprenorphine**

Degradable microspheres (MS 3 & MS4, duration buprenorphine elution of 10 and 30 days, respectively) loaded with buprenorphine (target loading of 2 or 4 mg/mL) were implanted subcutaneously in the dorsum of New-Zealand rabbit (n=5) without anesthesia to better observe the occurrence of a possible sedative effect. Injectable buprenorphine (Bupaq®, Multidose 0.3 mg/mL Injectable solution injectable, Virbac) was administrated at the dose of 60 $\mu$g/kg/day as reference. Blood samples (-2.5 mL) were through an auricular artery to assess the systemic concentration of buprenorphine at the following time points: pre-injection (T0), 15min, 30min, 1h, 3h, 5h, 24h, 2d, 4d, 7d, 14d, 21d, 28d. BPN quantifications were done by coupled HPLC-MS/MS (LOD = 0.1 ng/mL, LOQ = 0,3 ng/mL). The dose of buprenorphine injected was determined as the dose equivalent to repeated injectable buprenorphine injections over the period of buprenorphine elution form degradable microspheres, *i.e.* for a delivery of 10 days, the total dose injected with injectable BPN would be 30 or 60 $\mu$g/kg per injection X 10 injections.

Table 7: Animal groups for pharmacokinetics study. * pool of 2 pellets loaded at 3.2 and 3.3 mg/mL

| Animals | MS used | Buprenorphine loading (mg/ml) | Duration of in vitro buprenorphine release (days) | Time Equivalent Treatment (days) | Daily equivalence BPN ($\mu$g/kg/Day) | Total dose of BPN injected (mg) |
|---|---|---|---|---|---|---|
| **1** | MS4 | 6.5* | 30 | 30 | 60 | 6.7 |
| **2** | MS4 | 3 | 30 | 10 | 60 | 2.2 |
| **3** | MS3 | 2.8 | 10 | 10 | 60 | 2.1 |
| **4** | MS3 | 3 | 10 | 10 | 60 | 2.5 |
| **5** | MS3 | 1.5 | 10 | 10 | 30 | 1.3 |

The different doses administered to rabbits were chosen to allow for comparison of the animals by pairs with one parameter varying between the 2 animals: according to the MS degradation time and the amount of BPN injected.

Animals 1 and 2 received MS4 loaded at 3 mg/mL, but animal 1 received three times as much MS compared to animal 2, equivalent to a duration of treatment of 30 days and 10 days, respectively. Animals 2 and 3 received a similar amount of MS loaded at the same concentration, but animal 2 received MS4 while animal 3 received MS3. Animals 3 and 4 received the same treatment. Animals 4 and 5 both received MS3, but animal 4 received MS at 3 mg/mL and animal 5 received MS loaded at 1.5 mg/mL. The plasmatic levels were analyzed according to the MS degradation time and the BPN dose (Figure 4).

In vivo, after subcutaneous implantation in rabbit of buprenorphine loaded MS, the plasmatic passage of buprenorphine occurs without burst and last for 4 or 7 days for the MS3 and MS4, respectively, showing an effect of microsphere degradation time on *in vivo* buprenorphine elution (Table 8).

Table 8: Summary of selected pharmacokinetic values for sustained-release buprenorphine administered subcutaneously to rabbit.

| | T max | T last |
|---|---|---|
| **Free buprenorphine** | 30 min | 5 h |
| **MS3** | 5 h | 96 h |
| **MS4** | 24 h | 168 h |

Increasing the amount of BPN implanted for MS3 and MS4 did not increase the duration of plasma presence, but only the plasma concentration of buprenorphine rise.

**Example 6. Tissular concentration of buprenorphine at the implantation site one month after injection of buprenorphine loaded microspheres.**

One month after implantation, residual buprenorphine at implantation site was measured for all animals. In tissues, ng of BPN per gram were secondary expressed in concentration (nM).

Buprenorphine was detected in the skin of injection sites one month after injection. The residual concentration in tissue depends of the microsphere degradation time and the amount of implanted drug. Otherwise, the residual local concentrations, between 1.2 and 54 nM, could still be active to inhibit locally the mu receptors of buprenorphine ($IC_{50} \approx$ 1 nM) (Khanna & Pillarisetti. 2015. J Pain Res. 8:859-70).

**Example 7 : Size distribution of sterile hydrophilic microspheres (MS3 & MS4) after swelling in saline**

The sterile microspheres from the two batches used for the pharmacokinetic study have similar size distribution (50-120 $\mu$m) (See Figures 6A and 6B).

**Example 8: Histology of MS3 implantion in rabbit in several tissues. Histology (HES staining) was performed after 2 days, 4 days and 7 days.**

The hydrophilic degradable microsphere (MS3) was injected in rabbit in 5 different tissues in order to analyse the degradation and the inflammatory reaction triggers by degradation. 100 $\mu$L of bland (no buprenorphine loading) were injected in the muscle (saddle), derm, knee joint cavity, gum and the conjunctiva. Two rabbits were sacrified at day 2, day 4 and day 7. After formalin fixation, The samples were dehydrated in a series of alcohols, set in xylene and embedded in paraffin. Sections of 3-4 $\mu$m in thickness were cut and mounted on a microscope slide. After rehydratation in a series of alcohols, sections were stained with haematein-eosin-saffron (HES). Stained sections were scanned with the Nanozoomer device (HAMAMATSU) at x20 magnification. Analysis of the scans was done on a computer screen with a viewing software (NDP View) that replicates the functions of a microscope Histology of MS3 implants in the different tissues at day 2, 4 and 7 is shown in Figure 7 **(Fig. 7A: MS 3 after** Subconjonctival injection ; Fig. 7B: MS3 after intra-articular (joint knee) injection; Fig. 7C: MS3 after intra-muscular injection ; Fig. 7D: MS3 after injection in the gum).
Evolution of MS degradation and local inflammatory reaction in the different tissues is shown in Figure 9 and 10, respectively.

## Claims

**1.** A composition comprising an effective amount of buprenorphine or a pharmaceutically acceptable salt thereof, at

least one hydrophilic degradable microsphere comprising a crosslinked matrix, and a pharmaceutically acceptable carrier for administration by injection, the crosslinked matrix being based on at least:

a) between 10 % and 90 % of hydrophilic monomer of general formula (I):

$$(CH_2{=}CR_1)\text{-}CO\text{-}D \qquad (I)$$

wherein:

- D is O-Z or NH-Z, Z being $(C_1\text{-}C_6)$alkyl group, $-(CR_2R_3)_m\text{-}CH_3$, $-(CH_2\text{-}CH_2\text{-}O)_m\text{-}H$, $(CH_2\text{-}CH_2\text{-}O)_m\text{-}CH_3$, $-C(R_4OH)m$ or $-(CH_2)_m\text{-}NR_5R_6$ with m being an integer from 1 to 30;
- $R_1$, $R_2$, $R_3$, $R_4$, $R_5$ and $R_6$ are, independently of one another, hydrogen atom or a $(C_1\text{-}C_6)$alkyl group;

b) between 0.1 and 30% mol of a cyclic monomer having an exo-methylene group of formula (II):

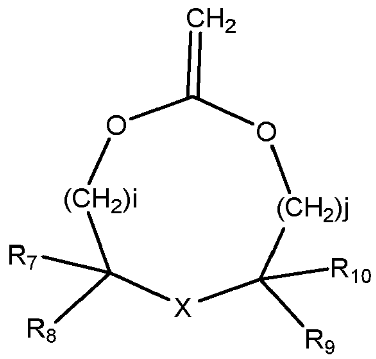

(II)

wherein:

- $R_7$, $R_8$, $R_9$ and $R_{10}$ are, independently of one another, hydrogen atom, a $(C_1\text{-}C_6)$alkyl group or an aryl group;
- i and j are independently of one another an integer chosen between 0 and 2; and
- X is a single bond or an oxygen atom;

and

c) between 5 % and 90 % of one degradable block copolymer cross-linker, wherein the degradable block copolymer crosslinker is linear or star-shaped and presents $(CH_2{=}(CR_{11}))$-groups at all its extremities, each $R_{11}$ being independently of one another hydrogen atom or a $(C_1\text{-}C_6)$alkyl group, and wherein the degradable block copolymer crosslinker has a partition coefficient P of between 0.50 and 11.20, or a hydrophobic/hydrophilic balance R between 1 and 20;

the percentages of the monomers a) to c) being expressed in moles relative to the total number of moles of the monomers.

**2.** The composition of claim 1, wherein the degradable block copolymer cross-linker c) is of general

$$(CH_2{=}CR_{11})\text{-}CO\text{-}X_n\text{-}PEG_p\text{-}X_k\text{-}CO\text{-}(CR_{11}{=}CH_2) \qquad (IIIa);$$

$$(CH_2{=}CR_{11})\text{-}CO\text{-}O\text{-}X_n\text{-}PEG_p\text{-}X_k\text{-}O\text{-}CO\text{-}(CR_{11}{=}CH_2) \qquad (IIIb)$$

$$PEG_p\text{-}(X_n\text{-}O\text{-}CO\text{-}(CR_{11}{=}CH_2))_z \qquad (IIIc);$$

wherein:

- each $R_{11}$ is independently of one another hydrogen atom or a $(C_1\text{-}C_6)$alkyl group;
- X independently represents, PLA, PGA, PLGA, PCL or PLAPCL;
- n, k and p respectively represent the degree of polymerization of X, and PEG, n and k independently being integers from 1 to 150, and p being an integer from 1 to 100;

- z represent the number of arms of the PEG molecule being integers from 3 to 8.

**3.** The composition of claim 2, wherein the degradable block copolymer cross-linker c) is of general formula (IIIa) or (IIIb) or (IIIc) wherein X represents PCL or PLAPCL, advantageously X independently represents PCL.

**4.** The composition of any one of claims 1 to 3, wherein the degradable block copolymer cross-linker c) is selected from the group consisting of compounds of general formula (IIIa), (IIIb) or (IIIc), in particular (IIIa) or (IIIb), as defined above, wherein:

- X= PLA, n + k=12 and p=13;
- X= PLAPCL, n + k=10 and p=13;
- X = PLAPCL, n + k= 9 and p=13;
- X = PLAPCL, n + k= 8 and p=13;
- X = PCL; n + k = 8 and p=13;
- X =PCL, n + k = 10 and p=4; or
- X =PCL, n + k = 12 and p=2.

**5.** The composition of any one of claims 1 to 4, wherein the degradable block copolymer cross-linker c) is present in the reaction mixture in an amount of between 5% and 60 %, by mole, relative to the total number of moles of the monomers.

**6.** The composition of any one of claims 1 to 5, wherein the cyclic monomer b) is selected from the group consisting of 2-methylene-1,3-dioxolane, 2-methylene-1,3-dioxane, 2-methylene-1,3-dioxepane, 2-methylene-4-phenyl-1,3-dioxolane, 2-Methylene-1,3,6-Trioxocane and 5,6-benzo-2-methylene-1,3dioxepane.

**7.** The composition of any one of claims 1 to 6, wherein the hydrophilic monomer a) is selected from the group consisting of sec-butyl acrylate, n-butyl acrylate, t-butyl acrylate, t-butyl methacrylate, methylmethacrylate, N-dimethyl-aminoethyl(methyl)acrylate, N,N-dimethylaminopropyl-(meth)acrylate, t-butylaminoethyl (methyl)acrylate, N,N-diethylaminoacrylate, acrylate terminated poly(ethylene oxide), methacrylate terminated poly(ethylene oxide), methoxy poly(ethylene oxide) methacrylate, butoxy poly(ethylene oxide) methacrylate, acrylate terminated poly(ethylene glycol), methacrylate terminated poly(ethylene glycol), methoxy poly(ethylene glycol) methacrylate, butoxy poly(ethylene glycol) methacrylate.

**8.** The composition of any one of claims 1 to 7, wherein the crosslinked matrix of the hydrophilic degradable microsphere is further based on a chain transfer agent d).

**9.** The composition of any one of claims 1 to 8, comprising between 0.1 and 50 mg/ml of buprenorphine or a pharmaceutically acceptable salt thereof.

**10.** A composition as defined in any one of claims 1 to 9 for use for preventing and/or treating moderate to severe pain.

**11.** A composition as defined in any one of claims 1 to 9 for use for preventing and/or treating post-surgical pain, advantageously for locally treating post-surgical pain.

**12.** A composition as defined in any one of claims 1 to 9 for use for preventing and/or treating opioid use disorders.

**13.** A composition for use according to any one of claims 10 to 12, wherein the buprenorphine is released in a constant rate during a period of 1 day to 30 days, advantageously between 1 and 7 days.

**14.** A hydrophilic degradable microsphere for use for delivering an effective amount of buprenorphine or a pharmaceutically acceptable salt thereof to a subject in need thereof, the hydrophilic degradable microsphere being as defined in any one of claims 1 to 9.

**15.** Pharmaceutical kit comprising:

i) at least one hydrophilic degradable microsphere as defined in any one of claims 1 to 9 in association with a pharmaceutically acceptable carrier for administration by injection;
ii) an effective amount of buprenorphine or a pharmaceutically acceptable salt thereof; and

iii) optionally an injection device,

the hydrophilic degradable microsphere and the buprenorphine or a pharmaceutically acceptable salt thereof being packed separately.

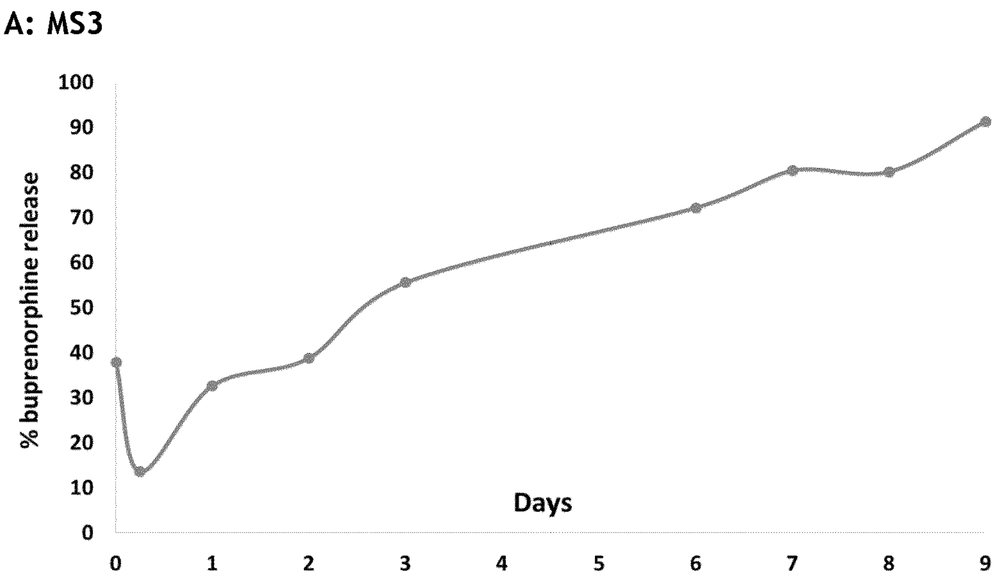
A: MS3
100
90
80
70
60
50
40
30
20
10
0
% buprenorphine release
Days
0
1
2
3
4
5
6
7
8
9

Fig. 1A

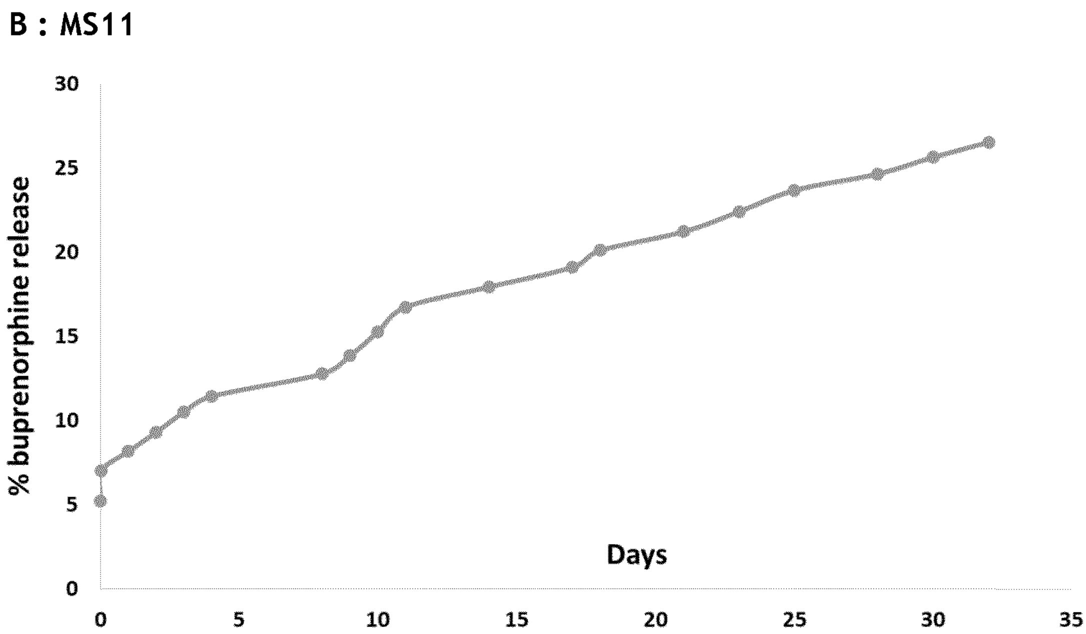
B : MS11
30
25
20
15
10
5
0
% buprenorphine release
Days
0
5
10
15
20
25
30
35

Fig. 1B

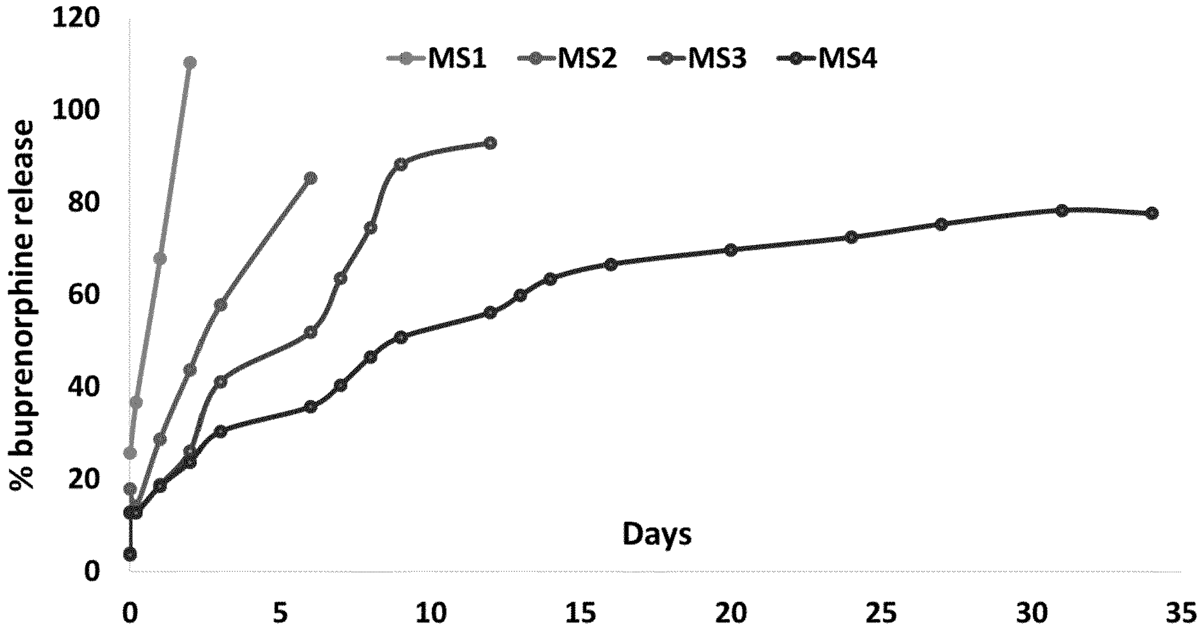
120
100
80
60
40
20
0
% buprenorphine release
MS1
MS2
MS3
MS4
Days
0
5
10
15
20
25
30
35

Fig. 2

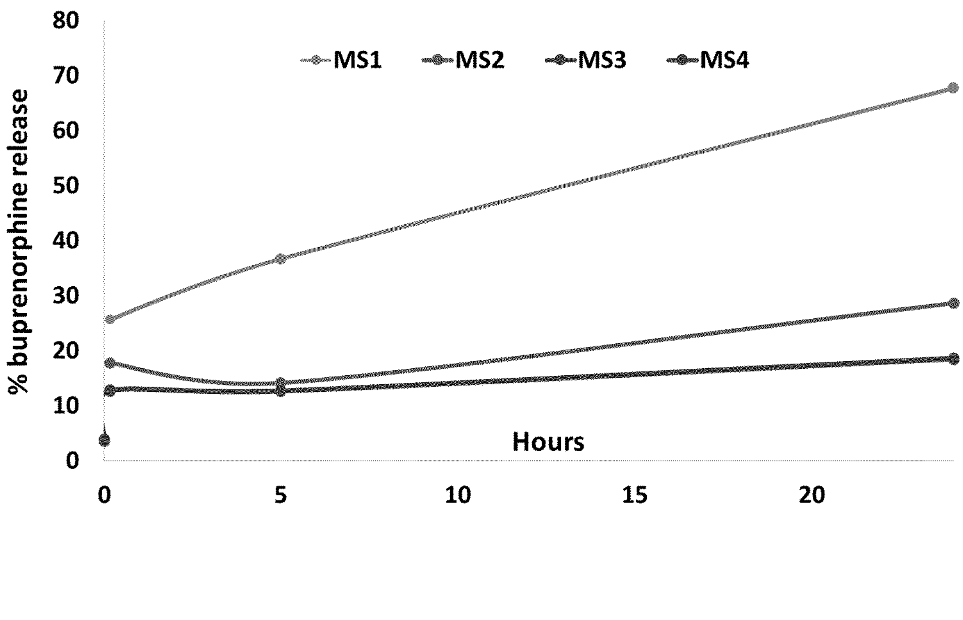
80
70
60
50
40
30
20
10
0
% buprenorphine release
MS1
MS2
MS3
MS4
Hours
0
5
10
15
20

Fig. 3

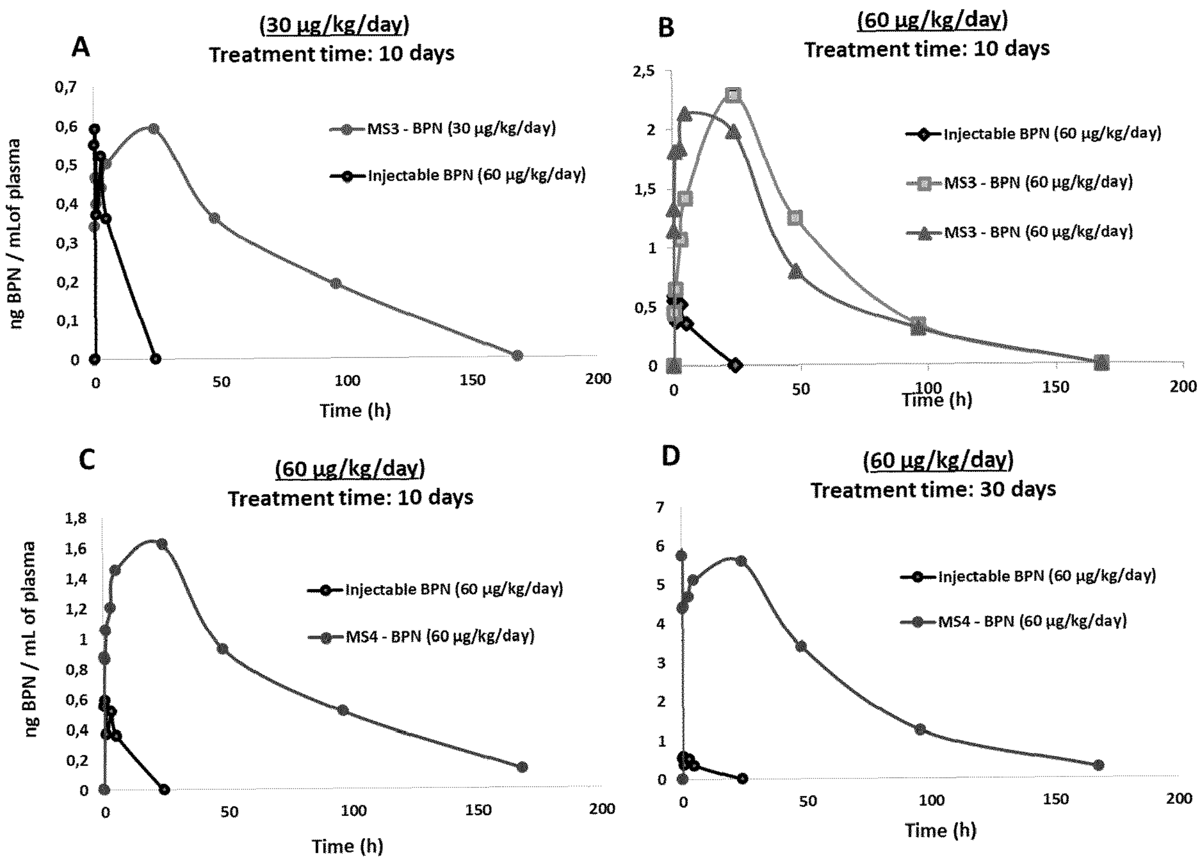
A
(30 µg/kg/day)
Treatment time: 10 days
ng BPN / mLof plasma
0,7
0,6
0,5
0,4
0,3
0,2
0,1
0
0
50
100
150
200
Time (h)
MS3 - BPN (30 µg/kg/day)
Injectable BPN (60 µg/kg/day)
B
(60 µg/kg/day)
Treatment time: 10 days
2,5
2
1,5
1
0,5
0
0
50
100
150
200
Time (h)
Injectable BPN (60 µg/kg/day)
MS3 - BPN (60 µg/kg/day)
MS3 - BPN (60 µg/kg/day)
C
(60 µg/kg/day)
Treatment time: 10 days
ng BPN / mL of plasma
1,8
1,6
1,4
1,2
1
0,8
0,6
0,4
0,2
0
0
50
100
150
200
Time (h)
Injectable BPN (60 µg/kg/day)
MS4 - BPN (60 µg/kg/day)
D
(60 µg/kg/day)
Treatment time: 30 days
7
6
5
4
3
2
1
0
0
50
100
150
200
Time (h)
Injectable BPN (60 µg/kg/day)
MS4 - BPN (60 µg/kg/day)

Fig. 4

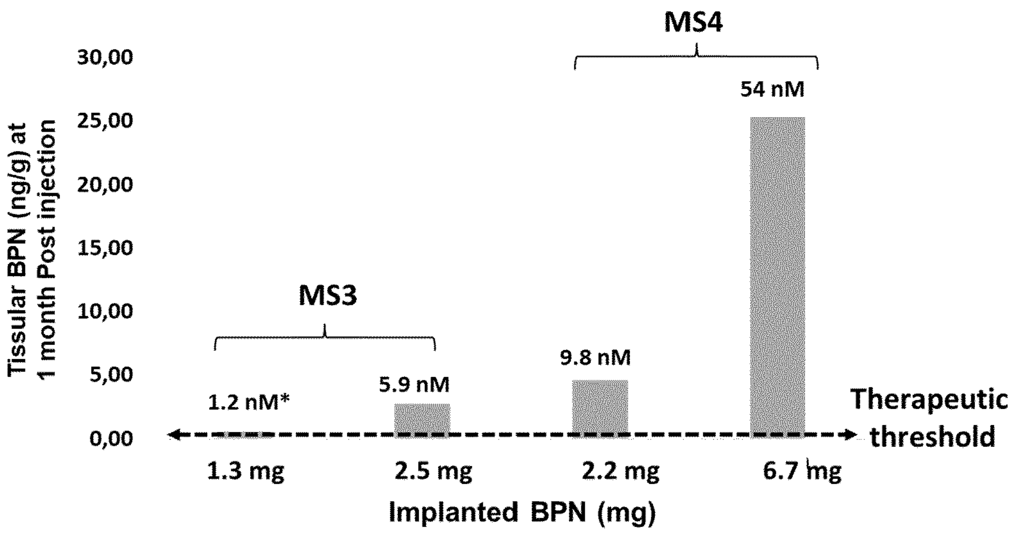
MS4
MS3
30,00
25,00
20,00
15,00
10,00
5,00
0,00
Tissular BPN (ng/g) at 1 month Post injection
1.2 nM*
5.9 nM
9.8 nM
54 nM
Therapeutic threshold
1.3 mg
2.5 mg
2.2 mg
6.7 mg
Implanted BPN (mg)

Fig. 5

MS3 :

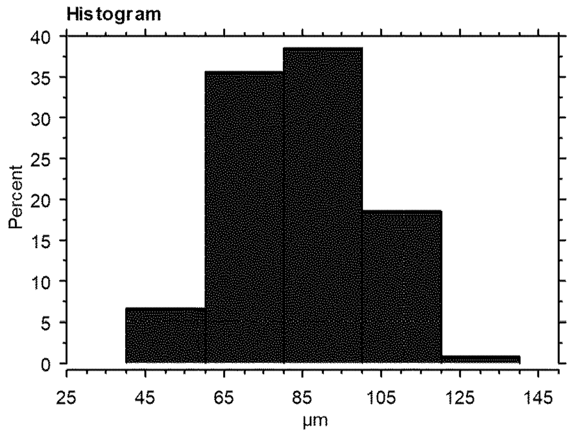


**Descriptive Statistics**

| | µm |
|---|---|
| Mean | 83,770 |
| Std. Dev. | 16,067 |
| Std. Error | 1,383 |
| Count | 135 |
| Minimum | 50,014 |
| Maximum | 122,096 |
| # Missing | 0 |

Fig. 6A

MS4 :

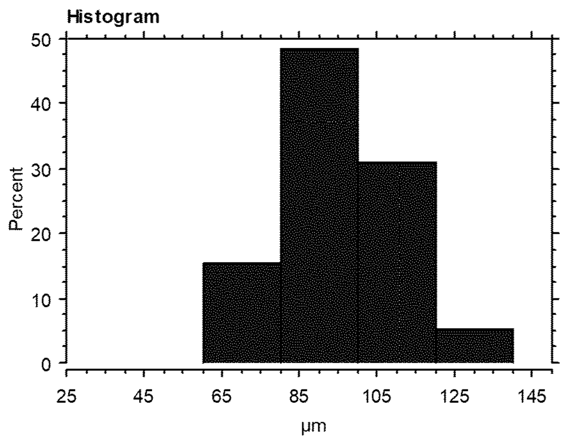


**Descriptive Statistics**

| | µm |
|---|---|
| Mean | 94,848 |
| Std. Dev. | 15,129 |
| Std. Error | 1,297 |
| Count | 136 |
| Minimum | 60,367 |
| Maximum | 123,685 |
| # Missing | 0 |

Fig. 6B

Subconjonctival injection of MS3 microspheres

Day 2

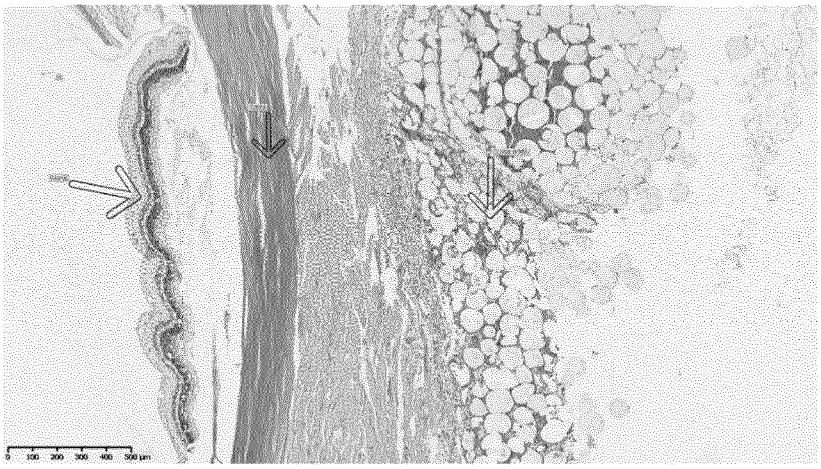

Day 4

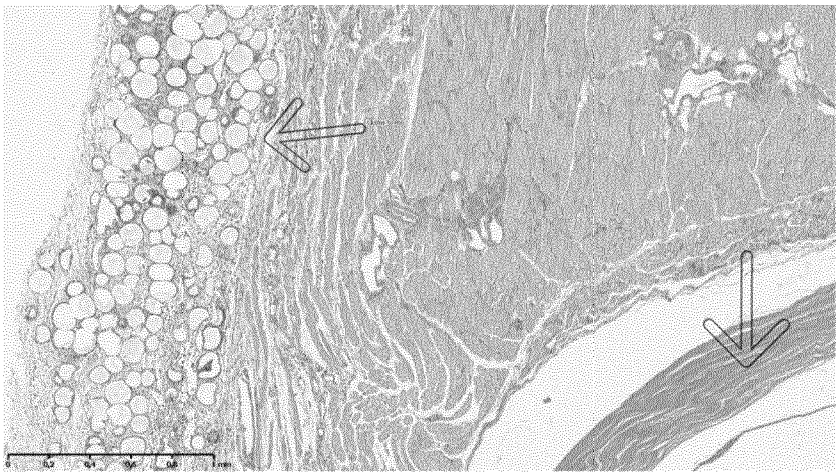

Day 7

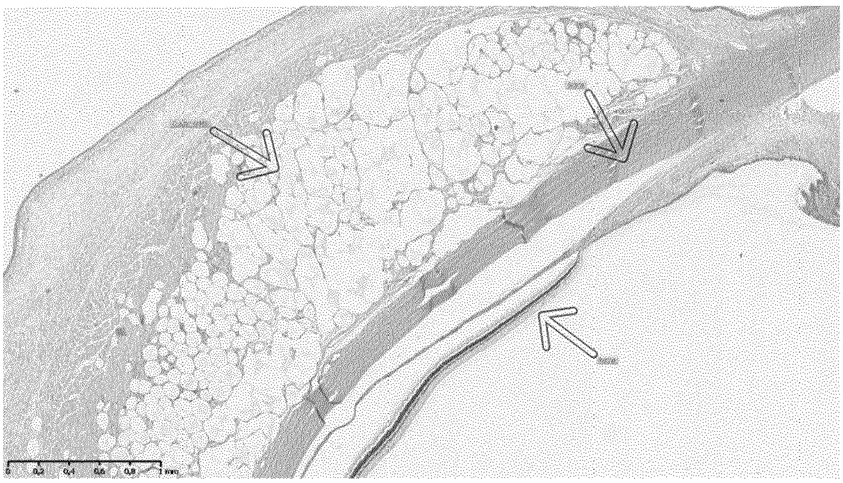

Fig. 7A

Intra-articular (joint knee) injection of MS3 microspheres

Day 4

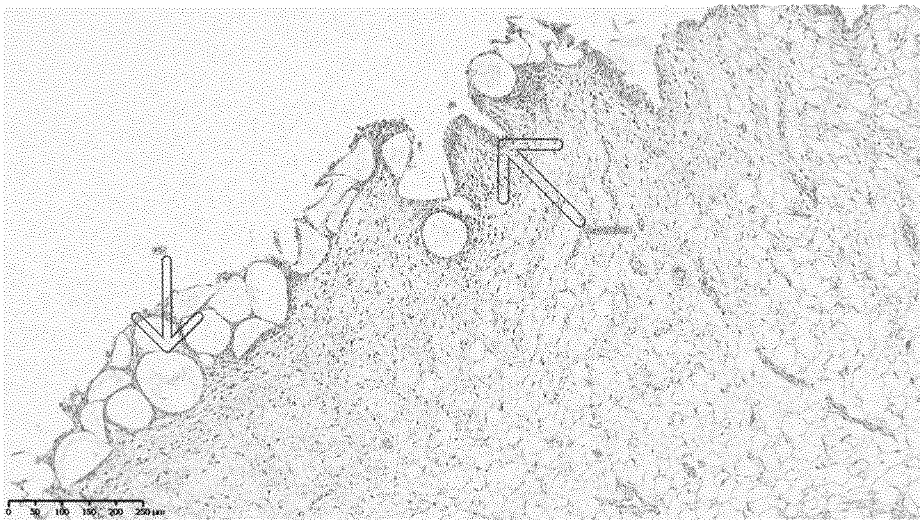

Day 7

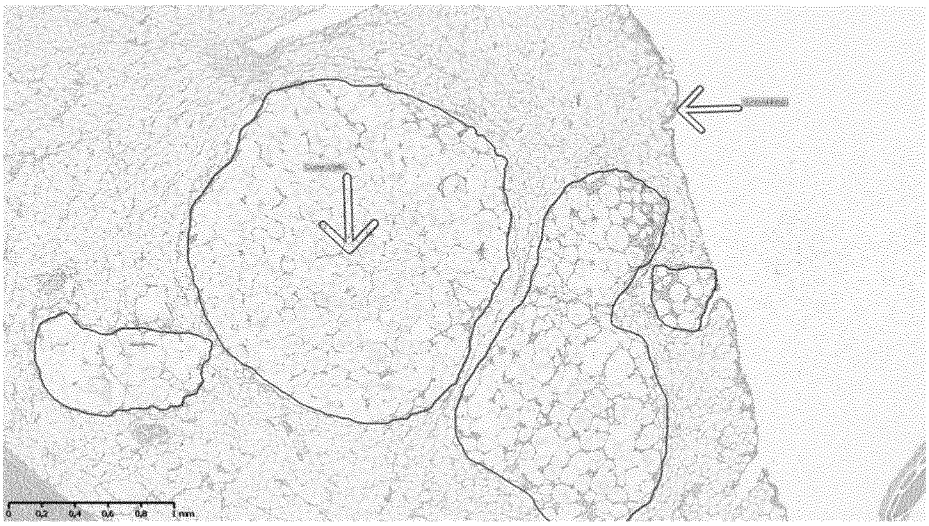

Fig. 7B

Muscle injection of MS3 microspheres

Day 2

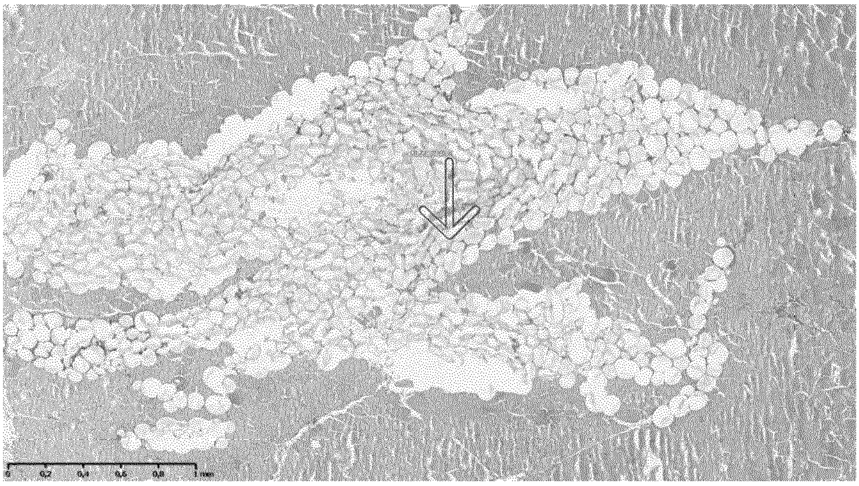

Day 4

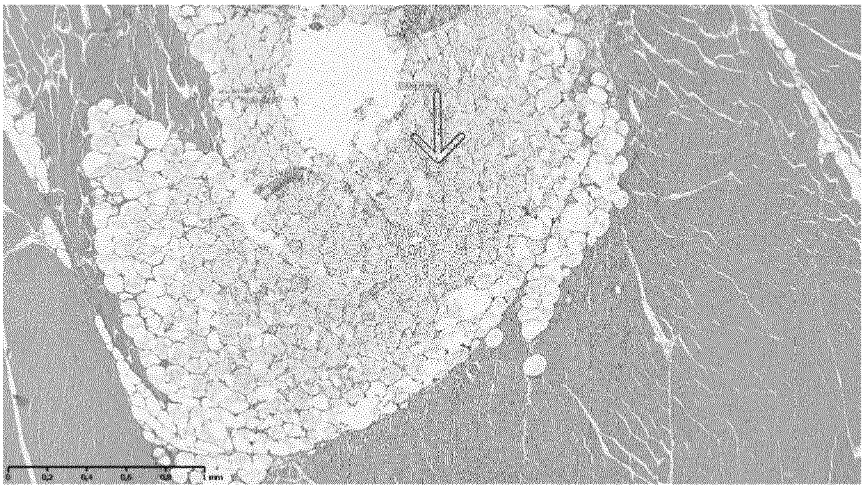

Day 7

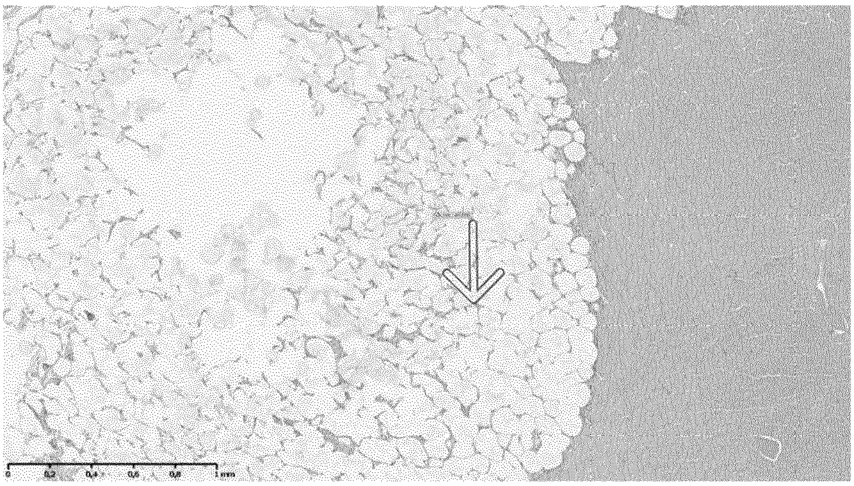

Fig. 7C

Gum injection of MS3 microspheres

Day 2

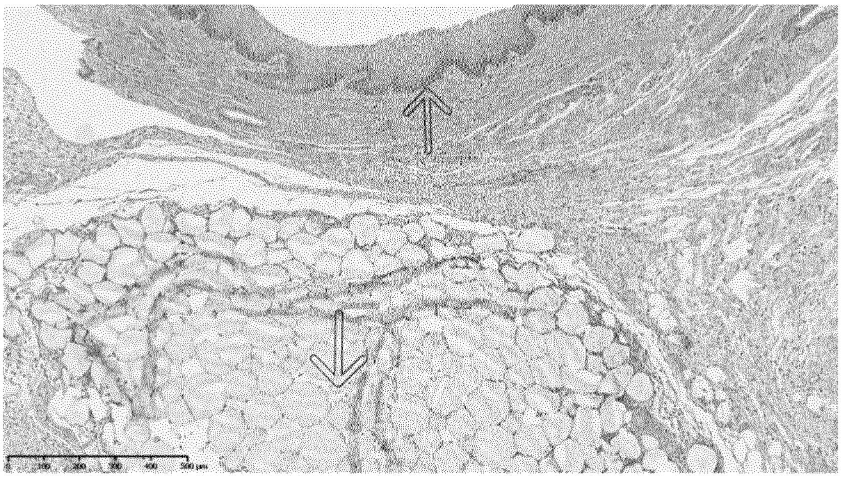

Day 4

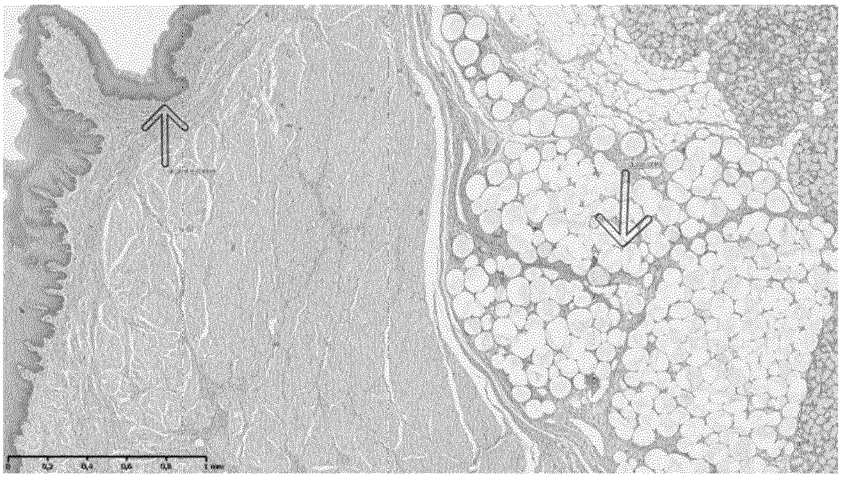

Day 7

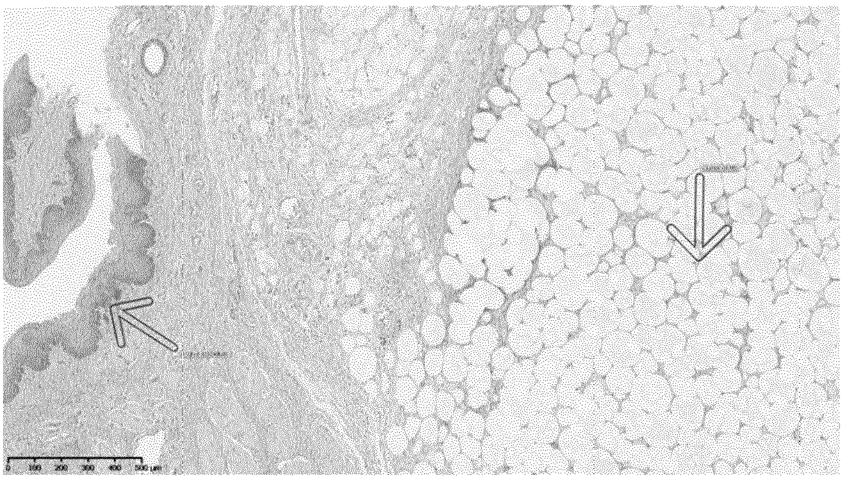

Fig. 7D

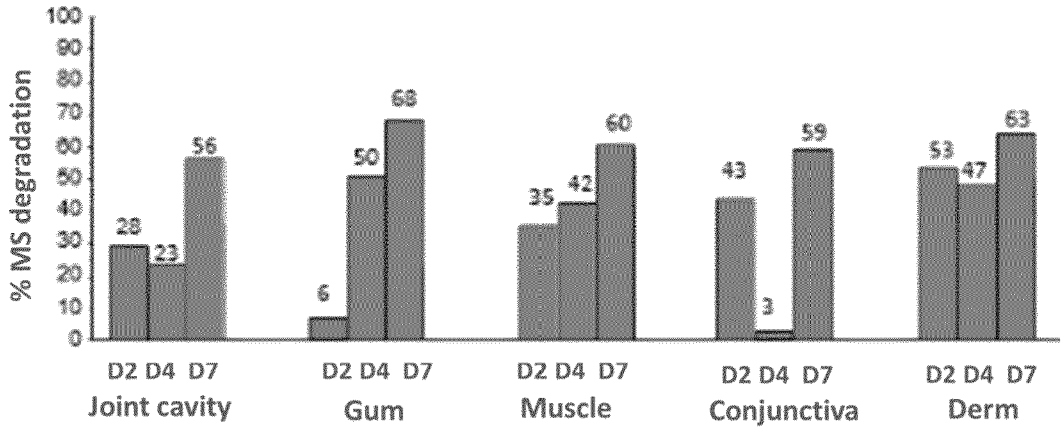
% MS degradation
100
90
80
70
60
50
40
30
20
10
0
28
23
56
6
50
68
35
42
60
43
3
59
53
47
63
D2 D4 D7
Joint cavity
D2 D4 D7
Gum
D2 D4 D7
Muscle
D2 D4 D7
Conjunctiva
D2 D4 D7
Derm

Fig. 8

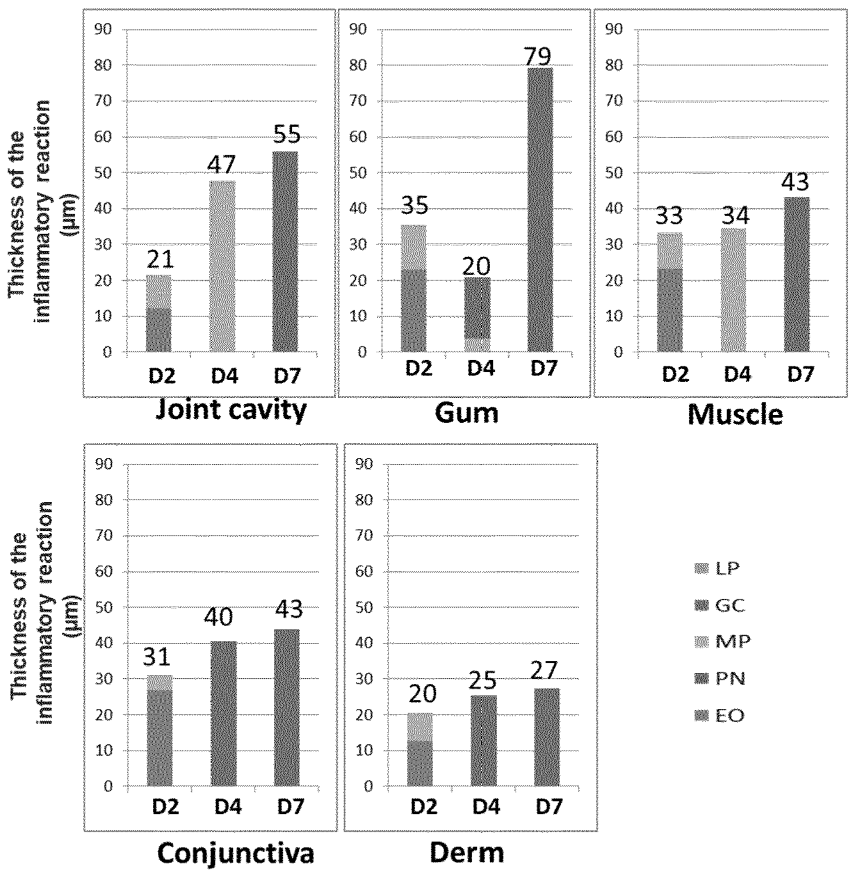
Thickness of the inflammatory reaction (µm)
90
80
70
60
50
40
30
20
10
0
21
47
55
D2
D4
D7
Joint cavity
35
20
79
Gum
33
34
43
Muscle
31
40
43
Conjunctiva
20
25
27
Derm
LP
GC
MP
PN
EO

Fig. 9

Europäisches Patentamt
European Patent Office
Office européen des brevets

# EUROPEAN SEARCH REPORT

Application Number
EP 20 30 5776

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 01/58447 A1 (EURO CELTIQUE SA [LU]; OSHLACK BENJAMIN [US]; CURTIS WRIGHT [US]) 16 August 2001 (2001-08-16) * example 20 * ----- | 1-15 | INV. A61K9/00 A61K9/16 A61K9/19 A61K9/50 |
| Y,D | WO 2017/147285 A1 (BIODELIVERY SCIENCES INT INC [US]) 31 August 2017 (2017-08-31) * paragraphs [0076], [0077] * ----- | 1-15 | |
| Y | US 2016/317453 A1 (RICHEY TRACY [US] ET AL) 3 November 2016 (2016-11-03) * example 4 * ----- | 1-15 | |
| Y | CN 105 596 298 A (UNIV SHANDONG) 25 May 2016 (2016-05-25) * abstract * ----- | 1-15 | |
| Y | WO 2012/120139 A1 (OCCLUGEL [FR]; CENTRE NAT RECH SCIENT [FR] ET AL.) 13 September 2012 (2012-09-13) * page 7, lines 14-19 * * page 24, lines 16-23 * * examples * * claims * ----- | 1-15 | TECHNICAL FIELDS SEARCHED (IPC): A61K |

The present search report has been drawn up for all claims

2

| Place of search | Date of completion of the search | Examiner |
|---|---|---|
| Munich | 9 February 2021 | Gerber, Myriam |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 30 5776

This annex lists the patent family members relating to the patent documents cited in the above-mentioned European search report. The members are as contained in the European Patent Office EDP file on
The European Patent Office is in no way liable for these particulars which are merely given for the purpose of information.

09-02-2021

| Patent document cited in search report | Publication date | Patent family member(s) | Publication date |
|---|---|---|---|
| WO 0158447 A1 | 16-08-2001 | AP 1665 A | 22-09-2006 |
| | | AT 431145 T | 15-05-2009 |
| | | AU 776666 B2 | 16-09-2004 |
| | | AU 776904 B2 | 23-09-2004 |
| | | BG 65828 B1 | 26-02-2010 |
| | | BR 0108379 A | 05-11-2002 |
| | | BR 0108380 A | 29-10-2002 |
| | | BR PI0108380 B1 | 01-10-2019 |
| | | CA 2400567 A1 | 16-08-2001 |
| | | CN 1418098 A | 14-05-2003 |
| | | CN 1423559 A | 11-06-2003 |
| | | CN 101703777 A | 12-05-2010 |
| | | CY 1109270 T1 | 02-07-2014 |
| | | CY 1114063 T1 | 27-07-2016 |
| | | CZ 20022706 A3 | 15-01-2003 |
| | | DK 1299104 T3 | 03-08-2009 |
| | | DK 2092936 T3 | 17-06-2013 |
| | | DK 2277521 T3 | 26-05-2015 |
| | | DK 2283842 T3 | 26-05-2015 |
| | | DK 2517710 T3 | 26-05-2015 |
| | | EA 200200840 A1 | 27-02-2003 |
| | | EE 200200437 A | 15-12-2003 |
| | | EP 1299104 A1 | 09-04-2003 |
| | | EP 2092936 A2 | 26-08-2009 |
| | | EP 2277521 A1 | 26-01-2011 |
| | | EP 2283842 A1 | 16-02-2011 |
| | | EP 2517710 A1 | 31-10-2012 |
| | | EP 3130338 A1 | 15-02-2017 |
| | | ES 2326730 T3 | 19-10-2009 |
| | | ES 2415407 T3 | 25-07-2013 |
| | | ES 2539904 T3 | 07-07-2015 |
| | | ES 2539945 T3 | 07-07-2015 |
| | | ES 2540103 T3 | 08-07-2015 |
| | | GE P20053614 B | 26-09-2005 |
| | | HK 1051487 A1 | 08-08-2003 |
| | | HK 1056822 A1 | 05-03-2004 |
| | | HK 1135907 A1 | 18-06-2010 |
| | | HU 0204163 A2 | 28-04-2003 |
| | | HU 0204229 A2 | 28-04-2003 |
| | | IL 151057 A | 20-03-2008 |
| | | IL 181356 A | 31-10-2011 |
| | | IL 204761 A | 31-10-2011 |
| | | JP 5351538 B2 | 27-11-2013 |
| | | JP 5676504 B2 | 25-02-2015 |
| | | JP 6063427 B2 | 18-01-2017 |
| | | JP 6403742 B2 | 10-10-2018 |

EPO FORM P0459

For more details about this annex : see Official Journal of the European Patent Office, No. 12/82

page 1 of 3

## ANNEX TO THE EUROPEAN SEARCH REPORT ON EUROPEAN PATENT APPLICATION NO. EP 20 30 5776

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2021

| Patent document cited in search report | Publication date | Patent family member(s) | Publication date |
|---|---|---|---|
| | | JP 2003522144 A | 22-07-2003 |
| | | JP 2003522146 A | 22-07-2003 |
| | | JP 2008019280 A | 31-01-2008 |
| | | JP 2009138007 A | 25-06-2009 |
| | | JP 2012158592 A | 23-08-2012 |
| | | JP 2012176993 A | 13-09-2012 |
| | | JP 2014169306 A | 18-09-2014 |
| | | JP 2015044838 A | 12-03-2015 |
| | | JP 2016222730 A | 28-12-2016 |
| | | JP 2017048210 A | 09-03-2017 |
| | | KR 20020071032 A | 11-09-2002 |
| | | KR 20020073542 A | 26-09-2002 |
| | | ME 00398 B | 10-05-2011 |
| | | MX PA02007686 A | 27-03-2003 |
| | | MX PA02007690 A | 23-08-2004 |
| | | NO 324717 B1 | 03-12-2007 |
| | | NZ 520554 A | 26-08-2005 |
| | | OA 12215 A | 09-05-2006 |
| | | PL 210845 B1 | 30-03-2012 |
| | | PL 358088 A1 | 09-08-2004 |
| | | PT 1255547 E | 24-11-2008 |
| | | PT 1299104 E | 21-07-2009 |
| | | PT 2092936 E | 20-06-2013 |
| | | PT 2277521 E | 01-07-2015 |
| | | PT 2283842 E | 01-07-2015 |
| | | PT 2517710 E | 01-07-2015 |
| | | SI 1299104 T1 | 31-10-2009 |
| | | SI 2092936 T1 | 31-07-2013 |
| | | SI 2277521 T1 | 31-07-2015 |
| | | SI 2283842 T1 | 31-07-2015 |
| | | SI 2517710 T1 | 31-07-2015 |
| | | SK 11342002 A3 | 04-03-2003 |
| | | TW I292317 B | 11-01-2008 |
| | | UA 79069 C2 | 25-05-2007 |
| | | US 2003143269 A1 | 31-07-2003 |
| | | US 2004092542 A1 | 13-05-2004 |
| | | US 2004186121 A1 | 23-09-2004 |
| | | US 2005095291 A1 | 05-05-2005 |
| | | US 2005181046 A1 | 18-08-2005 |
| | | US 2006039970 A1 | 23-02-2006 |
| | | US 2011097404 A1 | 28-04-2011 |
| | | US 2012288565 A1 | 15-11-2012 |
| | | US 2013251789 A1 | 26-09-2013 |
| | | US 2014099369 A1 | 10-04-2014 |
| | | US 2015182467 A1 | 02-07-2015 |
| | | US 2015231086 A1 | 20-08-2015 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 30 5776

This annex lists the patent family members relating to the patent documents cited in the above-mentioned European search report. The members are as contained in the European Patent Office EDP file on
The European Patent Office is in no way liable for these particulars which are merely given for the purpose of information.

09-02-2021

| Patent document cited in search report | Publication date | Patent family member(s) | Publication date |
|---|---|---|---|
| | | US 2017119685 A1 | 04-05-2017 |
| | | US 2018042859 A1 | 15-02-2018 |
| | | US 2019350868 A1 | 21-11-2019 |
| | | WO 0158447 A1 | 16-08-2001 |
| | | WO 0158451 A1 | 16-08-2001 |
| | | YU 58902 A | 28-11-2005 |
| WO 2017147285 A1 | 31-08-2017 | AU 2017223606 A1 | 13-09-2018 |
| | | CA 3015488 A1 | 31-08-2017 |
| | | CN 109069416 A | 21-12-2018 |
| | | EP 3419596 A1 | 02-01-2019 |
| | | JP 2019510008 A | 11-04-2019 |
| | | US 2017239240 A1 | 24-08-2017 |
| | | US 2019262333 A1 | 29-08-2019 |
| | | US 2020352935 A1 | 12-11-2020 |
| | | WO 2017147285 A1 | 31-08-2017 |
| US 2016317453 A1 | 03-11-2016 | NONE | |
| CN 105596298 A | 25-05-2016 | NONE | |
| WO 2012120139 A1 | 13-09-2012 | AU 2012224525 A1 | 26-09-2013 |
| | | EP 2683753 A1 | 15-01-2014 |
| | | ES 2601211 T3 | 14-02-2017 |
| | | JP 6063876 B2 | 18-01-2017 |
| | | JP 2014511427 A | 15-05-2014 |
| | | US 2013344160 A1 | 26-12-2013 |
| | | WO 2012120139 A1 | 13-09-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017147285 A

### Non-patent literature cited in the description


- **KAPIL et al.** *J Pain Symptom Manage,* 2013, vol. 46, 65-75
- **AL-TAWIL et al.** *Eur J Clin Pharmacol.,* 2013, vol. 69, 143-149
- **METHA et al.** *J Anaesthesiol Clin Pharmacol,* 2011, vol. 27, 211-4
- **KALSO et al.** *Pain,* 2002, vol. 98, 269-75
- **VARRASSI et al.** *Acta Anaesthesiol Scand.,* 1999, vol. 43, 51-5
- **RUSSELL.** *Neurosci Lett,* 1987, vol. 23, 107-12
- **STEIN et al.** *N Engl J Med.,* 1991, vol. 325, 1123-6
- **MACHELSKA ; CELIK.** *Frontiers in Pharmacology,* 2018, vol. 9
- **NIELSEN et al.** *Acta Anaesthesiol Scand.,* 2015, vol. 59, 830-45
- **ZHOU et al.** *J Pharmacol Exp Ther.,* 1998, vol. 286, 1000-6
- **TEGON et al.** *Tech. Coloproctol,* 2009, vol. 13, 219-24
- **ROSENTHAL ; GORADIA.** *Drug Des Devel Ther.,* 2017, vol. 11, 2493-2505
- **SIMON et al.** *Addiction.,* 2006, vol. 101, 420-32
- **WHITE et al.** *Drug Alcohol Depend.,* 2009, vol. 103, 37-43
- **NASSER et al.** *Clin Pharmacokinet,* 2014, vol. 53, 813-24
- **FROST et al.** *Addiction,* 2019, vol. 114, 1416-26
- **FOLEY et al.** *J Am Assoc Lab Anim Sci.,* 2011, vol. 50, 198-204
- **KHANNA ; PILLARISETTI.** *J Pain Res.,* 2015, vol. 8, 859-70